# EUROPEAN PATENT APPLICATION

(11) **EP 2 757 157 A1**
(43) Date of publication of application: **23.07.2014**
(21) Application number: 13151635.3
(22) Date of filing: 17.01.2013
(51) Int. Cl.: C12N 15/113, A61K 31/713, A61P 7/06, A61P 43/00

(54) **Modulation of mitophagy and use thereof**

(71) Applicant: Ecole Polytechnique Federale de Lausanne (EPFL), 1015 Lausanne (CH)
(72) Inventor: Trono, Didier, 1134 Vufflens-le-Château (CH); Barde, Isabelle, 01220 Divonne les Bains (FR)
(74) Representative: reuteler & cie SA

(57) **Abstract**

The present invention relates to an agent or composition comprising at least one of: (i) a micro-RNA targeting a mitophagy-associated gene or a variant thereof, (ii) a vector comprising a nucleic acid encoding said micro-RNA or variant thereof, (iii) an inducer of said micro-RNA, and (iv) an inhibitor of said micro-RNA, for use as a medicament. In particular, the invention relates to substances and compositions useful in the treatment of mitochondria-related diseases, in a subject, or to enhance protocols of *in vitro* production of red blood cells.

SEQUENCE LISTING

<110>  Ecole Polytechnique Federale de Lausanne (EPFL)

<120>  Modulation of mitophagy

<130>  P1539

<160>  20

<170>  PatentIn version 3.5

<210>  1
<211>  24
<212>  RNA
<213>  Homo sapiens

<400>  1
ucccugagac ccuuuaaccu guga                                              24


<210>  2
<211>  22
<212>  RNA
<213>  Homo sapiens

<400>  2
ucccugagac ccuaacuugu ga                                                22


<210>  3
<211>  24
<212>  RNA
<213>  Mus musculus

<400>  3
ucccugagga gcccuuugag ccug                                              24


<210>  4
<211>  24
<212>  RNA
<213>  Mus musculus

<400>  4
ucccugagac ccuuuaaccu guga                                              24


<210>  5
<211>  22
<212>  RNA
<213>  Mus musculus

<400>  5
uauagggauu ggagccgugg cg                                                22


<210>  6
<211>  23
<212>  RNA
<213>  Mus musculus

<400>  6
ucuggcuccg ugucuucacu ccc                                               23

<210>  7
<211>  22
<212>  RNA
<213>  Mus musculus

<400>  7
uccagcauca gugauuuugu ug                                                22


<210>  8
<211>  21
<212>  RNA
<213>  Mus musculus

<400>  8
ccugcuguaa gcuguguccu c                                                 21


<210>  9
<211>  22
<212>  RNA
<213>  Mus musculus

<400>  9
cacgcucaug cacacaccca ca                                                22


<210>  10
<211>  22
<212>  RNA
<213>  Mus musculus

<400>  10
auauacauac acacaccuac ac                                                22


<210>  11
<211>  22
<212>  RNA
<213>  Mus musculus

<400>  11
cauauacaua cacacaccua ca                                                22


<210>  12
<211>  23
<212>  RNA
<213>  Mus musculus

<400>  12
cauauacaua cacacacacg uau                                               23


<210>  13
<211>  554
<212>  PRT
<213>  Homo sapiens

<400>  13
   Met Ser Ala Asp Gly Gly Gly Ile Gln Asp Thr Gln Asp Lys Glu Thr
   1               5                   10                  15
   Pro Pro Glu Val Pro Asp Arg Gly His Pro His Gln Glu Met Pro Ser
               20                  25                  30
   Lys Leu Gly Glu Ala Val Pro Ser Gly Asp Thr Gln Glu Ser Leu His
           35                  40                  45
   Ile Lys Met Glu Pro Glu Glu Pro His Ser Glu Gly Ala Ser Gln Glu
       50                  55                  60
   Asp Gly Ala Gln Gly Ala Trp Gly Trp Ala Pro Leu Ser His Gly Ser
   65                  70                  75                  80
   Lys Glu Lys Ala Leu Phe Leu Pro Gly Gly Ala Leu Pro Ser Pro Arg
                   85                  90                  95
   Ile Pro Val Leu Ser Arg Glu Gly Arg Thr Arg Asp Arg Gln Met Ala
               100                 105                 110
   Ala Ala Leu Leu Thr Ala Trp Ser Gln Met Pro Val Thr Phe Glu Asp
           115                 120                 125
   Val Ala Leu Tyr Leu Ser Arg Glu Glu Trp Gly Arg Leu Asp His Thr
       130                 135                 140
   Gln Gln Asn Phe Tyr Arg Asp Val Leu Gln Lys Lys Asn Gly Leu Ser
   145                 150                 155                 160
   Leu Gly Phe Pro Phe Ser Arg Pro Phe Trp Ala Pro Gln Ala His Gly
                   165                 170                 175
   Lys Gly Glu Ala Ser Gly Ser Ser Arg Gln Ala Gly Asp Glu Lys Glu
               180                 185                 190
   Trp Arg Gly Ala Cys Thr Gly Ala Val Glu Val Gly Gln Arg Val Gln
           195                 200                 205
   Thr Ser Ser Val Ala Ala Leu Gly Asn Val Lys Pro Phe Arg Thr Arg
       210                 215                 220
   Ala Gly Arg Val Gln Trp Gly Val Pro Gln Cys Ala Gln Glu Ala Ala
   225                 230                 235                 240
   Cys Gly Arg Ser Ser Gly Pro Ala Lys Asp Ser Gly Gln Pro Ala Glu
                   245                 250                 255
   Pro Asp Arg Thr Pro Asp Ala Ala Pro Pro Asp Pro Ser Pro Thr Glu
               260                 265                 270
   Pro Gln Glu Tyr Arg Val Pro Glu Lys Pro Asn Glu Glu Glu Lys Gly
           275                 280                 285
   Ala Pro Glu Ser Gly Glu Glu Gly Leu Ala Pro Asp Ser Glu Val Gly
       290                 295                 300
   Arg Lys Ser Tyr Arg Cys Glu Gln Cys Gly Lys Gly Phe Ser Trp His
   305                 310                 315                 320
   Ser His Leu Val Thr His Arg Arg Thr His Thr Gly Glu Lys Pro Tyr
                   325                 330                 335
   Ala Cys Thr Asp Cys Gly Lys Arg Phe Gly Arg Ser Ser His Leu Ile
               340                 345                 350
   Gln His Gln Ile Ile His Thr Gly Glu Lys Pro Tyr Thr Cys Pro Ala
           355                 360                 365
   Cys Arg Lys Ser Phe Ser His His Ser Thr Leu Ile Gln His Gln Arg
       370                 375                 380
   Ile His Thr Gly Glu Lys Pro Tyr Val Cys Asp Arg Cys Ala Lys Arg
   385                 390                 395                 400
   Phe Thr Arg Arg Ser Asp Leu Val Thr His Gln Gly Thr His Thr Gly
                   405                 410                 415
   Ala Lys Pro His Lys Cys Pro Ile Cys Ala Lys Cys Phe Thr Gln Ser
               420                 425                 430
   Ser Ala Leu Val Thr His Gln Arg Thr His Thr Gly Val Lys Pro Tyr
           435                 440                 445
   Pro Cys Pro Glu Cys Gly Lys Cys Phe Ser Gln Arg Ser Asn Leu Ile
       450                 455                 460
   Ala His Asn Arg Thr His Thr Gly Glu Lys Pro Tyr His Cys Leu Asp
   465                 470                 475                 480
   Cys Gly Lys Ser Phe Ser His Ser Ser His Leu Thr Ala His Gln Arg
                   485                 490                 495
   Thr His Arg Gly Val Arg Pro Tyr Ala Cys Pro Leu Cys Gly Lys Ser
               500                 505                 510
   Phe Ser Arg Arg Ser Asn Leu His Arg His Glu Lys Ile His Thr Thr
           515                 520                 525
   Gly Pro Lys Ala Leu Ala Met Leu Met Leu Gly Ala Ala Ala Ala Gly
   530                 535                 540
   Ala Leu Ala Thr Pro Pro Pro Ala Pro Thr
   545                 550

<210>  14
<211>  500
<212>  PRT
<213>  Homo sapiens

<400>  14

Met Ala Pro Pro Ser Ala Pro Leu Pro Ala Gln Gly Pro Gly Lys Ala
1               5                   10                  15

Arg Pro Ser Arg Lys Arg Gly Arg Arg Pro Arg Ala Leu Lys Phe Val
            20                  25                  30

Asp Val Ala Val Tyr Phe Ser Pro Glu Glu Trp Gly Cys Leu Arg Pro
        35                  40                  45

Ala Gln Arg Ala Leu Tyr Arg Asp Val Met Arg Glu Thr Tyr Gly His
    50                  55                  60

Leu Gly Ala Leu Gly Cys Ala Gly Pro Lys Pro Ala Leu Ile Ser Trp
65                  70                  75                  80

Leu Glu Arg Asn Thr Asp Asp Trp Glu Pro Ala Ala Leu Asp Pro Gln
                85                  90                  95

Glu Tyr Pro Arg Gly Leu Thr Val Gln Arg Lys Ser Arg Thr Arg Lys
            100                 105                 110

Lys Asn Gly Glu Lys Glu Val Phe Pro Pro Lys Glu Ala Pro Arg Lys
        115                 120                 125

Gly Lys Arg Gly Arg Arg Pro Ser Lys Pro Arg Leu Ile Pro Arg Gln
    130                 135                 140

Thr Ser Gly Gly Pro Ile Cys Pro Asp Cys Gly Cys Thr Phe Pro Asp
145                 150                 155                 160

His Gln Ala Leu Glu Ser His Lys Cys Ala Gln Asn Leu Lys Lys Pro
                165                 170                 175

Tyr Pro Cys Pro Asp Cys Gly Arg Arg Phe Ser Tyr Pro Ser Leu Leu
            180                 185                 190

Val Ser His Arg Arg Ala His Ser Gly Glu Cys Pro Tyr Val Cys Asp
        195                 200                 205

Gln Cys Gly Lys Arg Phe Ser Gln Arg Lys Asn Leu Ser Gln His Gln
    210                 215                 220

Val Ile His Thr Gly Glu Lys Pro Tyr His Cys Pro Asp Cys Gly Arg
225                 230                 235                 240

Cys Phe Arg Arg Ser Arg Ser Leu Ala Asn His Arg Thr Thr His Thr
                245                 250                 255

Gly Glu Lys Pro His Gln Cys Pro Ser Cys Gly Arg Arg Phe Ala Tyr
            260                 265                 270

Pro Ser Leu Leu Ala Ile His Gln Arg Thr His Thr Gly Glu Lys Pro
        275                 280                 285

Tyr Thr Cys Leu Glu Cys Asn Arg Arg Phe Arg Gln Arg Thr Ala Leu
    290                 295                 300

Val Ile His Gln Arg Ile His Thr Gly Glu Lys Pro Tyr Pro Cys Pro
305                 310                 315                 320

Asp Cys Glu Arg Arg Phe Ser Ser Ser Ser Arg Leu Val Ser His Arg
                325                 330                 335

Arg Val His Ser Gly Glu Arg Pro Tyr Ala Cys Glu His Cys Glu Ala
            340                 345                 350

Arg Phe Ser Gln Arg Ser Thr Leu Leu Gln His Gln Leu Leu His Thr
        355                 360                 365

Gly Glu Lys Pro Tyr Pro Cys Pro Asp Cys Gly Arg Ala Phe Arg Arg
    370                 375                 380

Ser Gly Ser Leu Ala Ile His Arg Ser Thr His Thr Glu Glu Lys Leu
385                 390                 395                 400

His Ala Cys Asp Asp Cys Gly Arg Arg Phe Ala Tyr Pro Ser Leu Leu
                405                 410                 415

Ala Ser His Arg Arg Val His Ser Gly Glu Arg Pro Tyr Ala Cys Asp
            420                 425                 430

Leu Cys Ser Lys Arg Phe Ala Gln Trp Ser His Leu Ala Gln His Gln
        435                 440                 445

Leu Leu His Thr Gly Glu Lys Pro Phe Pro Cys Leu Glu Cys Gly Arg
    450                 455                 460

Cys Phe Arg Gln Arg Trp Ser Leu Ala Val His Lys Cys Ser Pro Lys
465                 470                 475                 480

Ala Pro Asn Cys Ser Pro Arg Ser Ala Ile Gly Gly Ser Ser Gln Arg
                485                 490                 495

Gly Asn Ala His
            500

<210>  15
<211>  505
<212>  PRT
<213>  Mus musculus

<400>  15

Met Ser Ala Asp Ser Gln Ser Ile Ala Ala Thr Glu Asn Glu Glu Lys
1               5                   10                  15

Ser His Glu Val Pro Gly Asn Val Gln His Cys Gly Asp Met Leu Ser
            20                  25                  30

Gly Gln Glu Glu Thr Val Pro Leu Gly Thr Ser Gln Glu Ser Thr His
        35                  40                  45

Ile Lys Ala Glu Pro Glu Glu Pro His Ser Glu Gly Ala Ser Arg Glu
    50                  55                  60

Asp Arg Thr Pro Gly Thr Gln Arg Trp Met Ser Pro Cys Pro Gly Pro
65                  70                  75                  80

Lys Asp Lys Val Val Pro Ser Pro Trp Thr Pro Val Leu Ser Arg Gly
                85                  90                  95

Gly Arg Thr Arg Glu Arg Lys Met Ala Ala Ala Leu Leu Thr Ala Trp
            100                 105                 110

Ser Gln Met Pro Val Thr Phe Glu Asp Val Ala Leu Tyr Leu Ser Gln
        115                 120                 125

Glu Glu Trp Gly Arg Leu Asp His Thr Gln Gln Asn Phe Tyr Arg Asp
    130                 135                 140

Val Leu Gln Gly Lys Asn Gly Leu Ala Leu Gly Ala Val Glu Met Gly
145                 150                 155                 160

Lys Val Val Pro Ala Leu Ala Val Ala Thr Leu Glu Asp Thr Lys Ser
                165                 170                 175

Ile Arg Thr Arg Ala Arg Trp Ala Pro Gly Glu Asp Pro Lys Cys Gly
            180                 185                 190

Gln His Val Ala Ser Gly Pro Gly Thr Lys Leu Thr Arg Asp Thr Gly
        195                 200                 205

Lys Ala Gly Gln Leu Lys Pro Ala Pro Ser Glu Ser Arg Pro Leu Lys
    210                 215                 220

Thr Pro Glu Asp Ser Gly Pro Glu Lys Pro Ser Glu Gly Glu Glu Ala
225                 230                 235                 240

Leu Lys Ser Gly Glu Glu Gly Leu Val Pro Asp Gly Asp Thr Gly Lys
                245                 250                 255

Lys Thr Tyr Lys Cys Glu Gln Cys Gly Lys Gly Phe Ser Trp His Ser
            260                 265                 270

His Leu Val Thr His Arg Arg Thr His Thr Gly Glu Lys Pro Tyr Thr
        275                 280                 285

Cys Thr Asp Cys Gly Lys Arg Phe Gly Arg Ser Ser His Leu Ile Gln
    290                 295                 300

His Gln Ile Ile His Thr Gly Glu Lys Pro Tyr Thr Cys Pro Ser Cys
305                 310                 315                 320

Trp Lys Ser Phe Ser His His Ser Thr Leu Ile Gln His Gln Arg Ile
                325                 330                 335

His Thr Gly Glu Lys Pro Tyr Val Cys Asp Arg Cys Ala Lys Arg Phe
            340                 345                 350

Thr Arg Arg Ser Asp Leu Val Thr His Gln Gly Thr His Thr Gly Ala
        355                 360                 365

Lys Pro His Lys Cys Pro Ile Cys Ser Lys Cys Phe Thr Gln Ser Ser
    370                 375                 380

Ala Leu Val Thr His Gln Arg Thr His Thr Gly Val Lys Pro Tyr Pro
385                 390                 395                 400

Cys Pro Glu Cys Gly Lys Cys Phe Ser Gln Arg Ser Asn Leu Ile Ala
                405                 410                 415

His Asn Arg Thr His Thr Gly Glu Lys Pro Tyr His Cys Leu Asp Cys
            420                 425                 430

Gly Lys Ser Phe Ser His Ser Ser His Leu Thr Ala His Gln Arg Thr
        435                 440                 445

His Arg Gly Val Arg Pro Tyr Ser Cys Pro Leu Cys Gly Lys Ser Phe
    450                 455                 460

Ser Arg Arg Ser Asn Leu His Arg His Glu Lys Ile His Thr Thr Gly
465                 470                 475                 480

Pro Lys Ala Leu Ala Met Leu Met Leu Gly Ala Ala Ala Ala Gly Ala
                485                 490                 495

Leu Thr Ala Pro Pro Pro Ala Ser Thr
            500                 505

<210>  16
<211>  500
<212>  PRT
<213>  Mus musculus

<400>  16

Met Ala Pro Pro Ser Ala Pro Leu Leu Leu Arg Ala Val Gly Glu Ala
1               5                   10                  15

Gly Pro Thr Arg Lys Arg Gly Arg Arg Pro Arg Ala Leu Lys Phe Val
            20                  25                  30

Asp Val Ala Val Tyr Phe Ser Ser Glu Glu Trp Gly Cys Leu Gln Pro
        35                  40                  45

Ala Gln Arg Thr Leu Tyr Arg Asp Val Met Arg Glu Thr Tyr Gly Leu
    50                  55                  60

Leu Gly Ala Leu Gly Cys Ala Gly Pro Lys Pro Ala Leu Ile Ser Trp
65                  70                  75                  80

Leu Glu Arg Asn Thr Asp Asp Trp Glu Pro Ala Ala Leu Asp Pro Gln
                85                  90                  95

Glu Tyr Arg Arg Trp Val Thr Phe Gln Arg Lys Thr Arg Ser Lys Gln
            100                 105                 110

Lys Thr Glu Glu Lys Asp Val Phe Pro Pro Lys Glu Ala Pro Arg Lys
        115                 120                 125

Gly Lys Arg Gly Arg Lys Pro Ser Lys Pro Arg Leu Ile Pro Arg Gln
    130                 135                 140

Thr Ser Gly Gly Pro Ile Cys Pro Asp Cys Gly Cys Thr Phe Pro Asp
145                 150                 155                 160

His Leu Ala Leu Glu Ser His Lys Cys Ala Gln Asn Leu Lys Lys Pro

                165                 170                 175

Tyr Pro Cys Pro Asp Cys Gly Arg Arg Phe Ser Tyr Pro Ser Leu Leu
            180                 185                 190

Val Ser His Arg Arg Ala His Ser Gly Glu Cys Pro Tyr Val Cys Asp
        195                 200                 205

Gln Cys Gly Lys Arg Phe Ser Gln Arg Lys Asn Leu Ser Gln His Gln
    210                 215                 220

Val Ile His Thr Gly Glu Lys Pro Tyr His Cys Pro Asp Cys Gly Arg
225                 230                 235                 240

Cys Phe Arg Arg Ser Arg Ser Leu Ala Asn His Arg Thr Thr His Thr
                245                 250                 255

Gly Glu Lys Pro His Gln Cys Pro Ser Cys Gly Arg Arg Phe Ala Tyr
            260                 265                 270

Pro Ser Leu Leu Ala Ile His Gln Arg Thr His Thr Gly Glu Lys Pro
        275                 280                 285

Tyr Thr Cys Leu Glu Cys Ser Arg Arg Phe Arg Gln Arg Thr Ala Leu
    290                 295                 300

Val Ile His Gln Arg Ile His Thr Gly Glu Lys Pro Tyr Pro Cys Pro
305                 310                 315                 320

Asp Cys Glu Arg Arg Phe Ser Ser Ser Ser Arg Leu Val Ser His Arg
                325                 330                 335

Arg Val His Ser Gly Glu Arg Pro Tyr Ala Cys Glu Asn Cys Glu Ala
            340                 345                 350

Arg Phe Ser Gln Arg Ser Thr Leu Leu Gln His Gln Leu Leu His Thr
        355                 360                 365

Gly Glu Lys Pro Tyr Pro Cys Pro Asp Cys Gly Arg Ala Phe Arg Arg
    370                 375                 380

Ser Gly Ser Leu Ala Ile His Arg Ser Thr His Thr Glu Glu Lys Leu
385                 390                 395                 400

His Ala Cys Asp Asp Cys Gly Arg Arg Phe Ala Tyr Pro Ser Leu Leu
                405                 410                 415

Ala Ser His Arg Arg Val His Ser Gly Glu Arg Pro Tyr Ala Cys Asp
            420                 425                 430

Leu Cys Ser Lys Arg Phe Ala Gln Trp Ser His Leu Ser Gln His Gln
      435                 440                 445

Leu Leu His Thr Gly Glu Lys Pro Phe Pro Cys Leu Glu Cys Gly Arg
    450                 455                 460

Cys Phe Arg Gln Arg Trp Ser Leu Ala Val His Lys Cys Cys Pro Asn
465                 470                 475                 480

Thr Pro Asn Gly Ser Pro Arg Pro Leu Ile Gly Gly Pro Ser Gln Arg
                485                 490                 495

Ser Ser Ala Leu
            500

<210>  17
<211>  1665
<212>  DNA
<213>  Homo sapiens

<400>  17
atgtctgcag acggcggagg catccaggac acccaggaca aggagacacc cccggaggtt      60

ccagatcgtg gacatcctca tcaggaaatg ccttctaagc tgggggaggc ggtaccttca     120

ggggacactc aggagtcact gcacattaag atggagcccg aagagccaca ctccgagggg     180

gcatcgcagg aggatggggc tcaaggtgcc tggggctggg cacccctaag tcacggctct     240

aaggagaaag ctctcttcct gcctggcgga gccctcccct ccccccggat ccccgtgctt     300

tcccgagagg ggaggaccag agaccggcag atggctgcag cgctcctcac tgcctggtcc     360

cagatgccag tgactttcga ggatgtggcc ttgtacctct cccgggagga gtggggacgg     420

ctggaccaca cgcagcagaa cttctacagg gatgtcctgc agaagaaaaa tgggctgtca     480

ctgggctttc ccttcagcag gcctttctgg gcccctcaag cgcacggcaa gggtgaggcc     540

tcgggctcca gccggcaggc aggagatgag aaggagtgga gaggcgcgtg cacaggagcc     600

gtcgaggtgg ggcagagggt gcagacctca tccgtggcag cccttgggaa tgtgaagccc     660

ttcagaacca gggcagggag agtccagtgg ggcgtcccgc agtgcgcgca ggaagcagcc     720

tgcggccgga gctcagggcc ggccaaagac tccgggcagc cggctgagcc agatcgcacc     780

ccggatgcag ctccgccaga ccccagtccc acggagcccc aggagtaccg cgtcccggag     840

aagcccaacg aggaggagaa gggcgccccg gagagtggcg aggagggcct ggcccctgac     900

agtgaggtgg gcaggaagag ctaccggtgc gagcagtgcg gcaagggctt cagctggcac     960

tcgcacctgg tgacgcaccg gcgcacgcac acgggcgaga agccctacgc ctgcactgac    1020

tgcgggaagc gcttcggccg cagctcgcac ctcatccagc accagatcat ccacacgggc    1080

gagaagccct acacctgccc cgcctgccgg aagagcttca gccaccactc cacgctgatt    1140

cagcaccagc gcatccacac cggagagaag ccctacgtgt gcgaccgctg cgccaagcgc 1200

ttcacccgcc gctcggactt ggtcacccac cagggcaccc acacgggcgc caagccgcac 1260

aagtgcccca tctgcgccaa gtgcttcacg cagagctcgg cgctagtcac ccaccagcgc 1320

acccacactg gggtcaagcc ctatccgtgc cccgagtgcg gcaagtgctt cagccagcgt 1380

tccaacctca tcgcgcacaa ccgcacacac acaggcgaga agccctacca ctgcctcgac 1440

tgcggcaaga gcttcagcca cagctcgcac ctcaccgcgc accagcgcac ccaccgtggc 1500

gtgcggccct acgcctgccc gttgtgcggc aagagcttca gccggcgctc caacctgcac 1560

cggcacgaga agatccacac caccgggccc aaggccctgg ccatgctgat gctgggggcg 1620

gcggcggcgg gggctctggc cacaccccca cccgctccca cctag 1665

<210> 18
<211> 1503
<212> DNA
<213> Homo sapiens

<400> 18
atggcgccac cttcggctcc gctccctgcg cagggaccag gaaaggccag acccagtcgg 60

aaaaggggca ggaggccgag ggctctgaag ttcgtggacg tggccgtgta cttctccccg 120

gaggagtggg gctgcctgcg gcccgcgcag agggccctgt accgggacgt gatgcgggag 180

acctacggtc acctgggcgc gctcgggtgc gcaggtccca aaccagccct catctcctgg 240

ttggaacgaa acaccgatga ctgggaaccg gctgctctag atccgcagga gtacccgaga 300

gggctaacag tccagagaaa aagcagaacc agaaagaaga atggggagaa ggaagtattc 360

ccgcctaagg aggcaccccg aaaggggaag cgaggccgga ggcccagcaa accccgactg 420

attcctaggc agacgtccgg gggccccatc tgccctgact gcggctgtac cttccctgat 480

catcaggccc tggagagcca caagtgcgcc cagaatctaa aaaagcctta cccttgccca 540

gactgtgggc gccgcttttc ctatccatcc ctgctggtca gtcaccggcg ggcacactcc 600

ggcgagtgcc cctatgtttg tgaccagtgt ggcaaacgtt tctcccagcg caagaacctc 660

tcccagcacc aggtcatcca tacaggggag aagccctatc actgccctga ctgtggtcgc 720

tgcttccgga ggagccggtc cttggccaat caccggacca cacacacagg tgaaaaaccc 780

caccagtgcc ctagctgtgg acgtcgcttc gcctacccct ccctgctagc catccaccag 840

cgtacacaca cgggagagaa gccctacact tgcctcgagt gcaaccgccg cttccgccag 900

cgcacggccc tcgtcatcca ccagcgcatc cacacgggcg agaagcccta cccgtgcccg 960

gactgcgagc ggcgcttctc ctcctcctct cgcctggtca gtcaccggcg tgtgcactct 1020

ggggagcgtc cctatgcctg cgagcactgt gaggcccgct tctcccagcg cagcacgctg 1080

ctccagcacc agctcttgca caccggagag aagccctacc cctgcccaga ctgtgggcgt 1140

gccttccggc ggagcggctc cctggccatc catcgcagca cgcacacaga ggagaagctg 1200

cacgcctgcg acgactgtgg tcgccgcttt gcctacccct cactgctggc cagccaccgg 1260

cgcgtgcact cgggcgagcg gccctatgcc tgcgaccttt gctccaagcg ttttgctcag 1320

tggagccacc tggcccagca ccagctgctg cacacggggg agaagccttt cccctgcctc 1380

gagtgtggcc ggtgcttccg ccagaggtgg tctctggctg tccacaagtg tagccccaag 1440

gccccaaact gtagccctag atctgctatc gggggctcca gtcagagggg caacgcccat 1500

tag 1503

<210> 19
<211> 1518
<212> DNA
<213> Mus musculus

<400> 19
atgtctgcag acagtcaaag catcgcggct actgagaacg aggagaaatc ccatgaggtc 60

ccaggtaatg tgcaacactg tggagacatg ctctctggac aggaggagac agtgcccttg 120

ggaacctctc aggagtcaac acacatcaag gcagagccag aagagccaca cagtgagggg 180

gcatcacggg aggacaggac tccaggaaca cagcgctgga tgtccccgtg tcctggccct 240

aaggacaaag ttgttccttc tccctggact cctgtactgt cccgaggggg acggaccaga 300

gagcgaaaga tggctgctgc actccttact gcctggtccc agatgccggt gacctttgag 360

gatgtggccc tgtatctctc ccaggaggag tggggaaggc tggaccacac acagcaaaat 420

ttctacagag atgtactaca ggggaaaaac gggctggctt tgggagccgt ggaaatgggg 480

aaggtggttc cagccttggc cgtcgcaacc ctagaggaca caaaatcgat aaggactagg 540

gccaggtggg ccccggggga agacccaaag tgtggtcagc acgtggctag tggcccaggt 600

acaaagctga ccagagacac tgggaaagct ggtcagctaa agccagcccc atctgaatca 660

cgtcctctca agaccccaga ggacagcggc ccagagaaac ccagtgaggg ggaggaggct 720

ctgaaaagtg gcgaagaggg tctggtaccc gacggtgaca cgggcaagaa gacatacaag 780

tgcgagcagt gtggcaaggg cttcagttgg cactcgcacc tggtgacaca ccggcgtacg 840

cacacgggcg agaagcctta tacctgcacg gactgtggca agcgcttcgg ccgcagctca 900

cacctcatcc agcaccagat catccacacg ggcgagaagc cctatacctg cccctcgtgt 960

tggaagagct tcagccacca ctccacgctg atccagcacc agcgcatcca cactggtgag 1020

aagccctacg tgtgcgaccg ctgtgccaag cgcttcaccc gtcgctcgga cctagtcaca 1080

caccagggca ctcacaccgg cgccaagccc cacaagtgcc ccatctgcag caagtgcttc 1140

acccagagct ccgccctggt cacgcaccag cgcacccaca ccggcgtcaa gccctacccg 1200

tgcccggagt gcggcaagtg cttcagccag cgctccaatc tcatcgcaca taatcgcacc 1260

cacacgggcg agaagcccta ccactgcctc gactgtggca agagcttcag ccacagctcg 1320

cacctcactg cccaccaacg cactcaccgt ggcgtgaggc cctactcctg cccactttgc 1380

ggcaagagct tcagccgccg ctccaacctg caccggcacg agaagatcca caccactgga 1440

cccaaggctc tggccatgct catgctgggg gcggcagctg cgggggcgct cacggcacca 1500

ccacctgctt ccacttag 1518

<210> 20
<211> 1503
<212> DNA
<213> Mus musculus

<400> 20
atggcgccac cttcggctcc gcttctcctg cgggcagtcg gagaggctgg acctactcgg 60

aaaaggggca ggaggccaag ggccttgaag ttcgtggacg tggccgtgta cttctcctcg 120

gaggagtggg ggtgtctgca gcccgctcag aggactctgt accgggatgt gatgcgcgag 180

acctacggcc tcctgggcgc gctcggatgc gcaggtccca aaccagctct catctcctgg 240

ttggaacgaa ataccgatga ctgggaacca gcagccctag atccgcagga gtaccggaga 300

tgggtaacat tccagagaaa aaccagatcc aagcagaaga ctgaagagaa ggacgtattc 360

ccacctaagg aagcaccccg aaaggggaaa cgaggcggaa agcccagcaa accccggctg 420

attcccaggc agacatccgg gggccccatc tgccctgact gtggctgtac ctttcctgac 480

cacctcgccc tggagagcca caagtgcgcc cagaatctga agaaacccta tccctgcccg 540

gattgtgggc gtcgcttttc ctacccatcc ctgttggtca gtcaccggcg ggcacactcc 600

ggtgagtgcc cttacgtttg tgaccagtgt ggcaaacgtt tttcccagag gaaaaacctg 660

tctcagcacc aggtcataca cacaggcgag aagccctacc actgccctga ctgtggccgc 720

tgctttcgca ggagccggtc gttggccaat caccggacca cgcacaccgg tgagaagcct 780

catcagtgcc ctagttgtgg gcgtcgcttc gcctatccat ctctgctcgc tatccaccaa 840

cggacacaca caggggagaa gccctatacc tgcctagagt gcagccgtcg cttccgccag 900

cgcaccgccc tggtcatcca ccagcgcatc catactggag agaagcctta cccatgtcct 960

gactgtgagc ggcgtttctc ctcctcctct cgcctggtta gccaccggcg tgtgcactct 1020

ggggagcggc cctatgcctg tgagaactgc gaagcccgct tttctcagcg cagcaccctg 1080

ctccagcacc agctcttgca cacaggcgag aaaccctacc cctgcccaga ctgtggacgt 1140

gccttccgga ggagcggctc cttggccatc catcgcagca cgcacacgga ggagaagctg 1200

cacgcctgcg atgactgcgg ccgccgcttt gcctatccct cactgctggc cagtcatcgg 1260

cgcgtgcact caggcgagcg gccctatgcc tgtgaccttt gctccaaacg ctttgcccag 1320

tggagccact tgtctcagca ccagcttctg cacactgggg aaaagccttt cccctgcctt 1380

gagtgtggcc ggtgcttccg ccagaggtgg tccctggctg tccacaagtg ctgccccaac 1440

acccccaatg gtagccctag acctcttata ggagggccca gccagaggag cagtgctctt 1500

tag 1503

## Description

### Field of the Invention

The present invention relates to substances and compositions thereof useful in the modulation of mitophagy. In particular, the invention relates to substances and compositions useful in the treatment of mitochondria-related diseases, in a subject.

### Background of the Invention

Mitophagy, which corresponds to a selective autophagy of mitochondria, is a process by which a cell regulates its number of mitochondria depending on its energetic needs. This process also constitutes an important mitochondrial quality control mechanism that eliminates damaged mitochondria.

Mitophagy also mediates removal of non-damaged mitochondria in cell differentiation processes such as maturation of red blood cells. Erythropoiesis releases about one hundred billion new red cells every day from the human adult bone marrow. This process is initiated by the differentiation of hematopoietic stem cells (HSC) into the earliest erythroid progenitor identified *ex vivo,* the slowly growing burst-forming unit-erythroid (BFU-E). This cell morphs into the rapidly dividing CFU-E (colony-forming unit-erythroid), the proliferation of which is stimulated by the hypoxia-induced hormone erythropoietin. Further differentiation occurs through a highly sophisticated program orchestrated by lineage- and stage-specific combinations of protein- and RNA-based transcription regulators (Lawrie, 2010, Br J Haematol 150, 144 *;* Hattangadi et al, 2011, Blood 118, 6258). It culminates in the elimination of all intracellular organelles including mitochondria and the nucleus to yield the fully mature erythrocyte, with on board some 250 million molecules of hemoglobin as almost sole cargo.

Mitophagy is a process important not only for red cell maturation, but also for destroying paternal mitochondria in fertilized oocytes, regulating lipid homeostasis, reducing the production of mitochondria-derived reactive oxygen species (ROS) in settings such as ischemic cardiomyocytes or acetaminophen-intoxicated hepatocytes, removing dysfunctional mitochondria as source of mtDNA mutations in aging cells, preventing neurodegeneration, minimizing inflammatory reactions and more generally alleviating mitochondria-induced tissue damage (Ding and Yin, 2012, Biol Chem 393, 547)*.*

In the case of red blood cells maturation, mitophagy has been partially characterized with the involvement of proteins like BNIP3L (BCL2/adenovirus E1B 19kDa interacting protein 3-like), GABARAP (GABA(A) receptor-associated protein-like 2), Atg8 (Autophagy-related protein 8), ULK1 (unc-51-like kinase 1) et Atg7 (Autophagy-related protein 7). However, much is still to be learned about the molecular mechanisms of these events in the red blood cells as well as in other systems, not only to understand more fully the cause of red cell disorders or more generally of mitophagy-related disorders but also to propose novel therapeutic approaches to these disorders and to facilitate the *in vitro* manufacturing of red blood cells, for instance for transfusion.

### Summary of the Invention

The present invention is directed towards agents for modulating mitophagy in a cell, compositions thereof, and uses thereof in therapy and diagnostic.

A first aspect of the invention provides an agent or composition comprising at least one of: (i) a micro-RNA targeting a mitophagy-associated gene or a variant thereof, (ii) a vector comprising a nucleic acid encoding said micro-RNA or variant thereof, (iii) an inducer of said micro-RNA, and (iv) an inhibitor of said micro-RNA, for use as a medicament.

In a second aspect, it is provided the use of said agent or composition in the manufacture of a medicament.

A third aspect of the invention relates to a pharmaceutical composition comprising an agent as described above.

A fourth aspect of the invention provides a method of modulating mitophagy in a subject, comprising administering said agent or composition to said subject.

A fifth aspect of the invention relates to a method of treating, preventing, or attenuating a mitochondria-related disease in a subject comprising administering said agent or composition to said subject.

A sixth aspect of the invention provides the use of said agent or composition for the manufacture of a medicament for treating, preventing, or attenuating a mitochondria-related disease.

A seventh aspect of the invention provides an *ex-vivo* method of prognosis and/or diagnosis of a mitochondria-related disease in a subject comprising determining, in a biological sample from said subject: a) the level of at least one of: (i) a micro-RNA targeting a mitophagy-associated gene, (ii) a KRAB-ZFP, (iii) a mitophagy-associated gene, and/or b) whether a nucleic acid comprising a mutation in at least one of (i) a micro-RNA targeting a mitophagy-associated gene, (ii) a gene encoding a KRAB-ZFP, (iii) the nucleic acid sequence of the DNA target of a KRAB-ZFP, (iv) a mitophagy-associated gene, is present.

An eighth aspect of the invention is a method of identifying a compound capable of modulating mitophagy comprising determining the level of at least one of said micro-RNA or at least one of said KRAB-ZFP.

A ninth aspect of the invention relates to a kit for carrying out any one of the methods and uses according to the invention.

A tenth aspect of the invention relates to a method for *in vitro* production of red blood cells comprising a step of inducing mitophagy in cells in culture by contacting said cells with an agent or composition comprising at least one inhibitor of a micro-RNA targeting a mitophagy-associated gene or a variant thereof.

Other features and advantages of the invention will be apparent from the following detailed description.

### Description of the figures

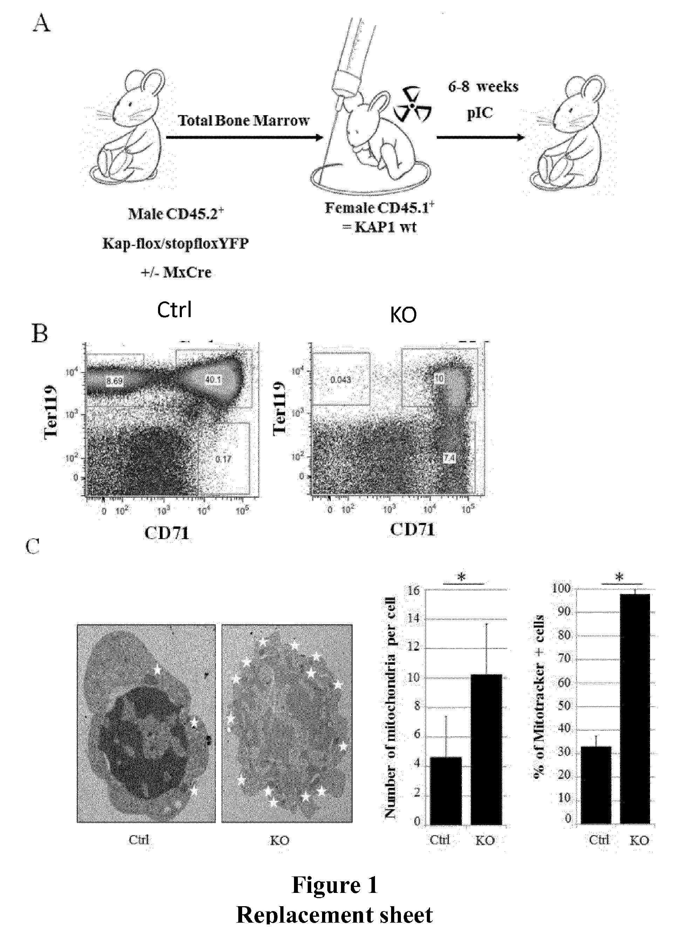

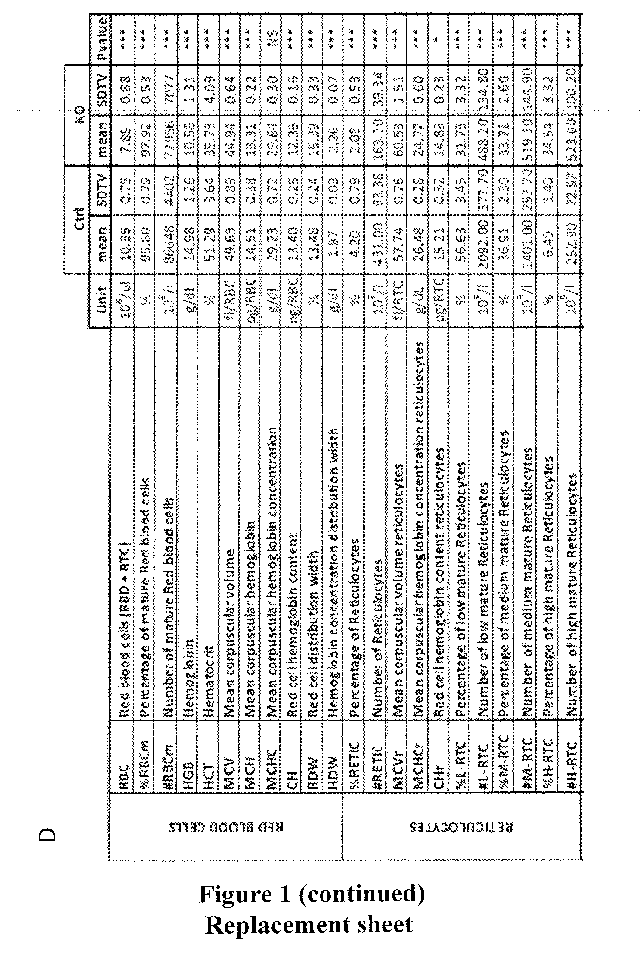

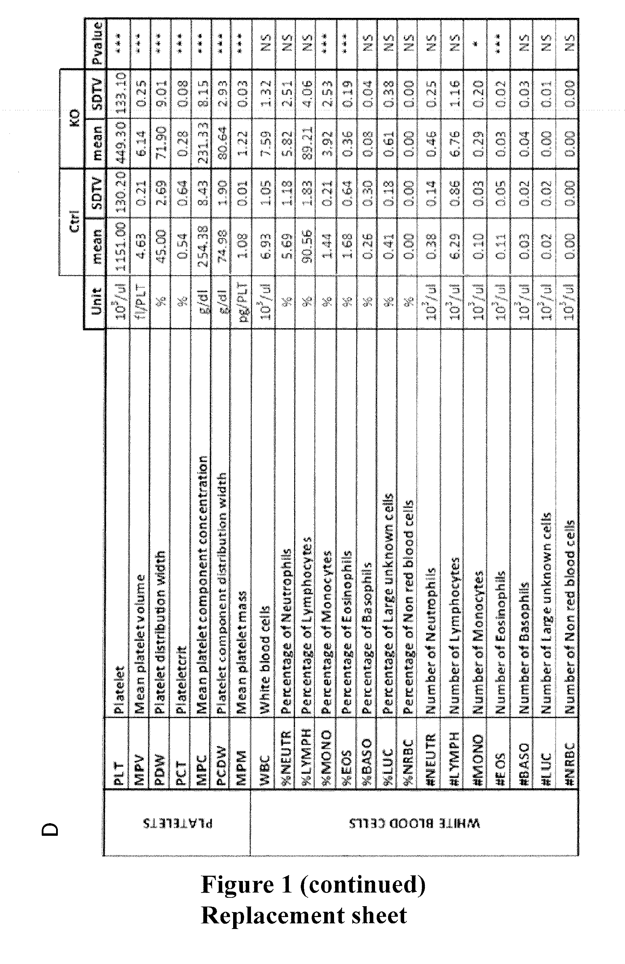

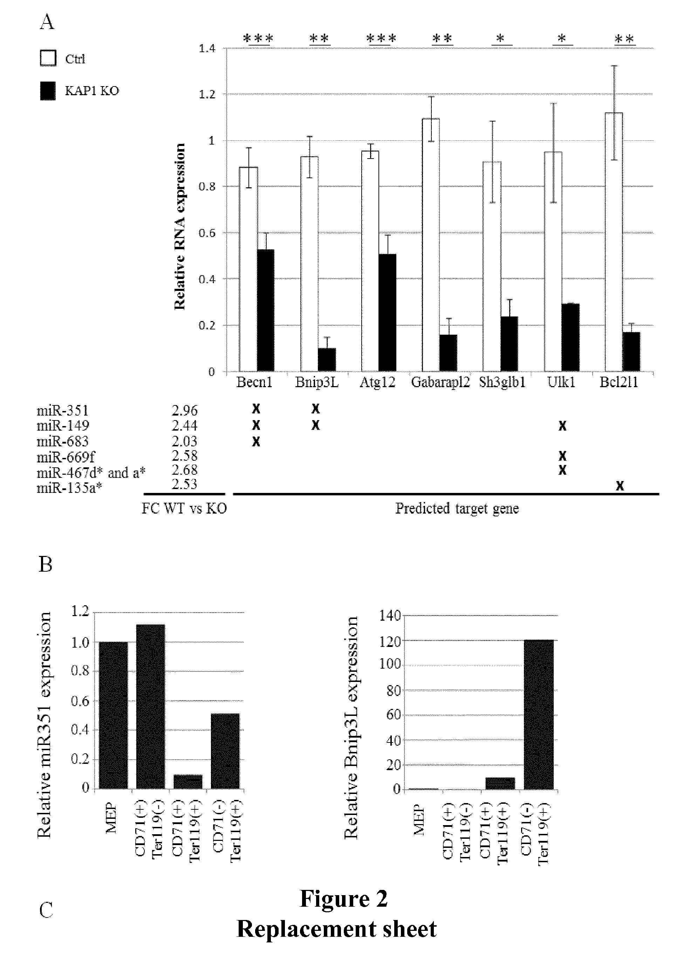

**Figure 1** **shows that mitophagy is defective in Kap1-deleted erythroblasts** (A) General strategy. Total bone marrow from Kap-flox/stopfloxYFP CD45.2 male mice, with or without a Mx-Cre transgene, was injected into lethally irradiated CD45.1 female mice, before pIC injection 6-8 weeks later to induce hemato-specific Kap1 knockout. (B) Blocked maturation of KAP-1 defective erythroblasts. FACS analysis of the total bone marrow 2 weeks after pIC injection, from control (Ctrl) and Kap1 KO mice, stained for CD71 and Ter119. (C) Increased mitochondrial content of Kap1-deleted erythroblasts. Bone marrow CD71+Ter119+ cells harvested from control and Kap1 KO mice 7 weeks post-pIC injections were analyzed by electron microscopy (left, with white stars next to mitochondria), counting the average number of mitochondria visualized for each cell (middle; n = 10, *p <0.05), and by Mitotracker staining (right, n=4,*p <0.05). (D) loss of KAP1 leads to a series of hematological abnormalities. **Figure 2** **shows that a KAP1-miRNA cascade controls red cell mitophagy.** (A) Deregulation of mitophagy-directed miRNAs and targets in Kap1 KO erythroblasts. Top, Decreased expression of mitophagy-associated genes in Kap1 KO erythroblasts, documented here by RT-QPCR (control -Ctrl- and KO mice, n=4, *p<0.05, **p<0.01, ***p<0.001). Bottom, fold-change (FC) of indicated miRNAs expression on same samples, probed with the Applied miRNA chip and confirmed by RTQPCR; predicted miRNA-target pairs are indicated by X. (B) miR351 and Bnip3L expression along erythroid differentiation, from the megakaryocyte/erythroid progenitor (MEP: Lin-Sca1-CD117+ CD34-CD16.32-) to proerythroblasts (CD71+Ter119-), basophilic erythroblasts (CD71+Ter119+) and orthochromatic erythroblasts (CD71-Ter119+). The normalized expression in MEP was set at 1.

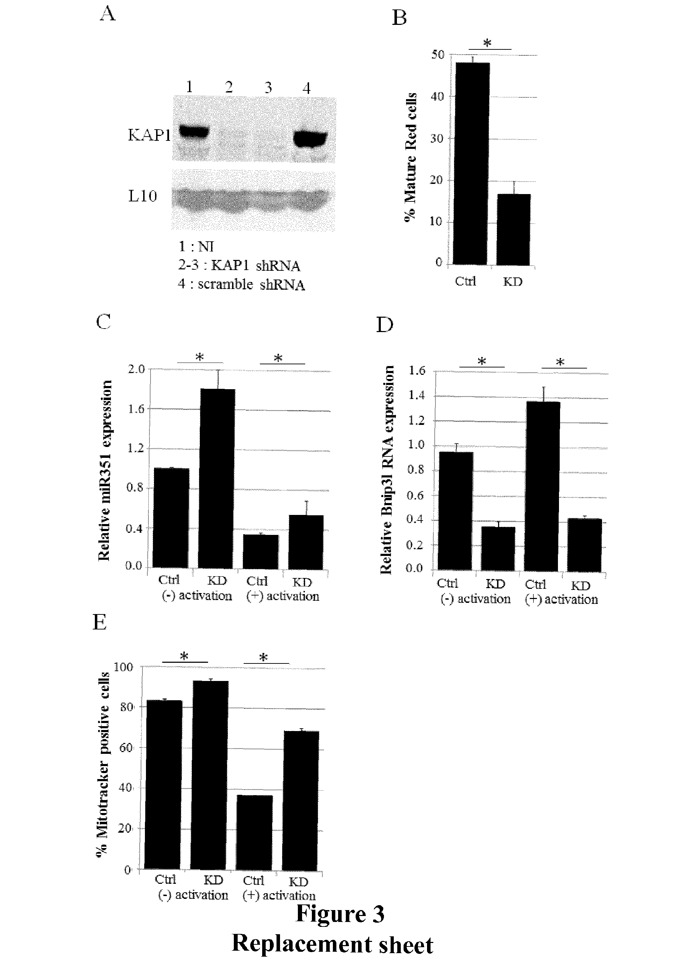

**Figure 3** **shows the KAP1-miR351-Bnip3L-mitophagy connection in MEL cells.** (A) Western blot analysis of MEL cells, illustrating efficient KAP1 depletion by a Kap1 knockdown (KD) shRNA-expressing lentiviral vector, compared to cells either non-infected (NI) or transduced with a scramble shRNA vector. The L10 ribosomal protein was used to normalize. (B) Impaired erythroid differentiation in Kap1 KD MEL cells. Differentiation was induced 3 days after transduction, and cells were examined three days later by benzidine staining and bright field microscopy (n=4, counting 100 cells for each condition). miR-351 (C) and Bnip3L (D) expression in indicated MEL cells/conditions. Normalized expression in control non-activated cells was set at 1. Controls were either non-transduced cells, or cells transduced with an empty or a scramble shRNA vector. (E) Mitochondrial content of MEL cells was assessed in parallel by Mitotracker staining (n=5, *p<0.05).

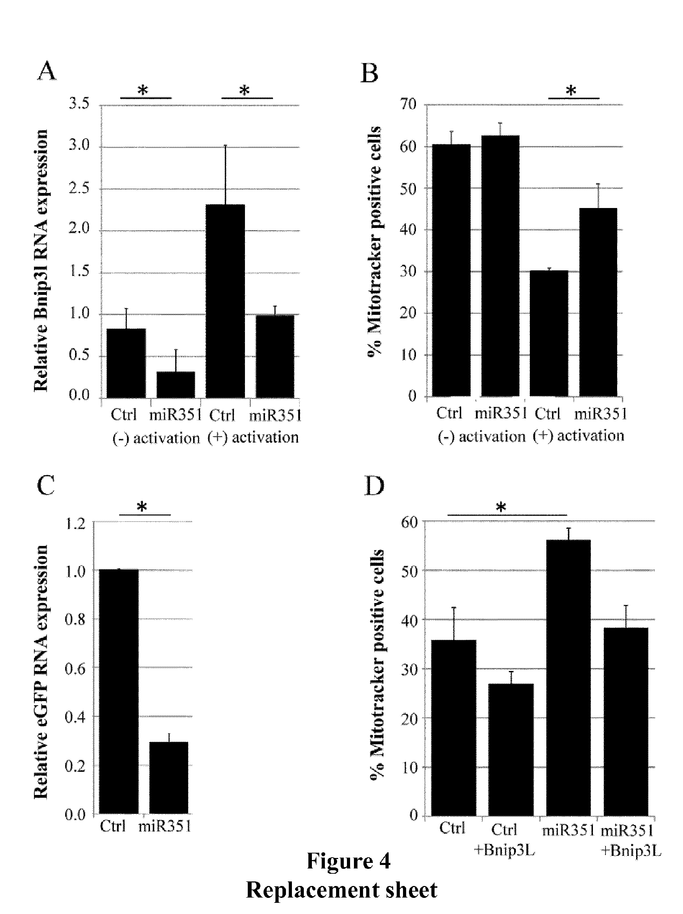

**Figure 4** **shows that miR-351 targets Bnip3L.** (A) RT-QPCR measurement of Bnip3L expression in control or miR351-overexpressing MEL cells (miR-351) induced or not to differentiate. Normalized expression in Ctrl (cells transduced with an empty vector and not activated) was set at 1. (B) Mitochondrial content of same cells, assessed by Mitotracker staining (n=5, *p<0.05). (C) miR-351 targets the Bnip3L 3'UTR. Control or miR-351-overexpressing MEL cells transduced with a GFP-expressing lentiviral vector bearing the Bnip3L 3'UTR. Fourteen days later GFP expression was checked by RT-QPCR. Ctrl, for which the normalized value was set at 1, was a combination of cells not overexpressing miR-351 and transduced with the GFP-expressing lentiviral vector with the Bnip3L 3'UTR, and cells overexpressing the miR-351 but transduced with the GFP-expressing lentiviral vector without this sequence (n=3, *p<0.05). (D)
Complementation of miR-351-overexpressing MEL cells with a lentiviral vector encoding Bnip3L devoid of its 3'UTR. Co-transduced cells were induced to differentiate and subjected to Mitotracker staining. Ctrl was cells transduced with an empty miRNA vector (n=3, *p<0.05).

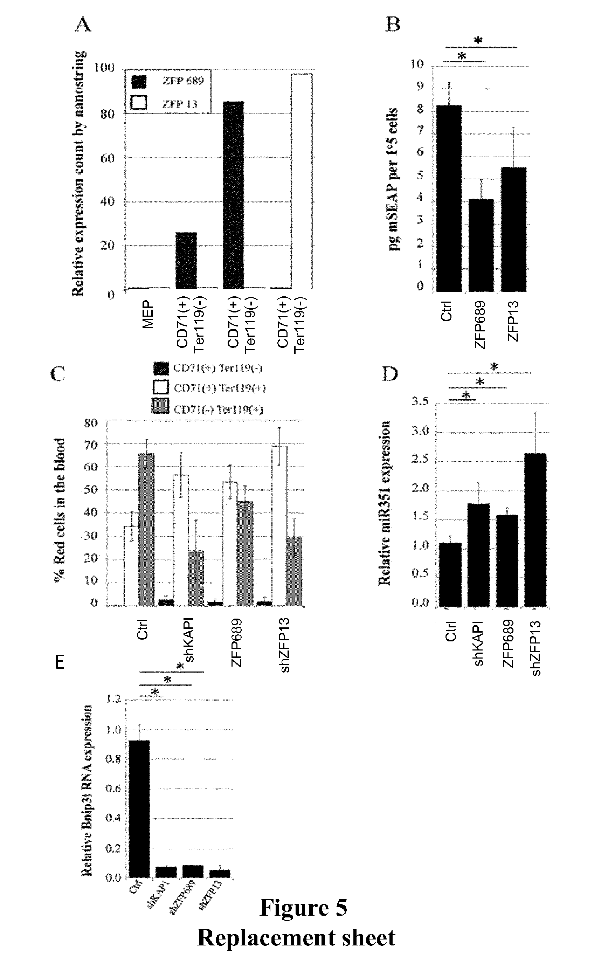

**Figure 5** **shows the erythroblast-specific KRAB-ZFPs control of the miR-351/Bnip3L/mitophagy axis.** (A) ZFP689 and ZFP13 are induced during erythroid differentiation. NanoString nCounter-based RNA quantification was performed on indicated cells isolated from wild-type mice. (B) ZFP689 and ZFP13 repress a vector carrying a miR-351-close KAP1-binding site. MEL cells were transduced with a lentiviral vector containing 500 bp from the X chromosome encompassing a miR351-close KAP1-binding site identified by Chipseq (see Fig. 2) cloned upstream of a human PGK promoter driving mSEAP. Five days later cells were re-transduced with ZFP689-or ZFP13-overexpressing lentiviral vectors, and SEAP was measured in the supernatant another 5 days later, normalizing results for cell numbers. Ctrl is a combination of ZFP overexpressing cells cotransduced with a vector without the KAP1-binding site and cells cotransduced with a LacZ-overexpressing vector with a vector carrying the KAP1-binding site (n=3, *p<0.05). (C-E) Hematopoietic-specific KD of ZFP689 or ZFP13 blocks red cell maturation and mitophagy. (C) CD45.2 LSK cells were transduced with GFP-expressing, empty or scramble-shRNA (Ctrl), KAP1-, ZFP689- or ZFP13-shRNA lentiviral vectors, engrafted into lethally irradiated CD45.1 mice, and erythroid differentiation was evaluated by FACS 8 wks later. The CD71+Ter119+, CD45.2+, eGFP+ population was then sorted and analyzed by RT-QPCR for miR351 (D) and Bnip3L (E) expression (n=6, *p<0.05).

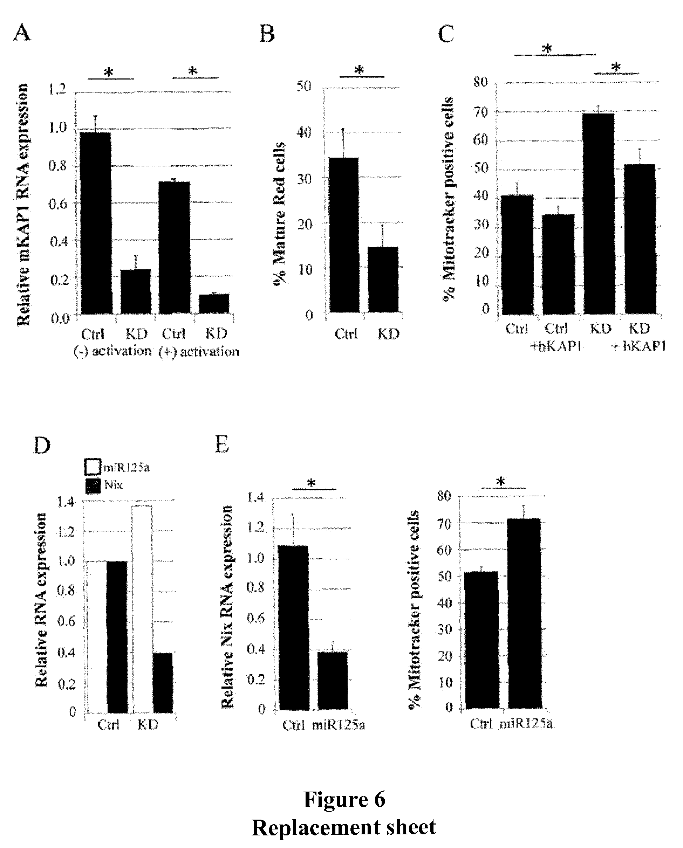

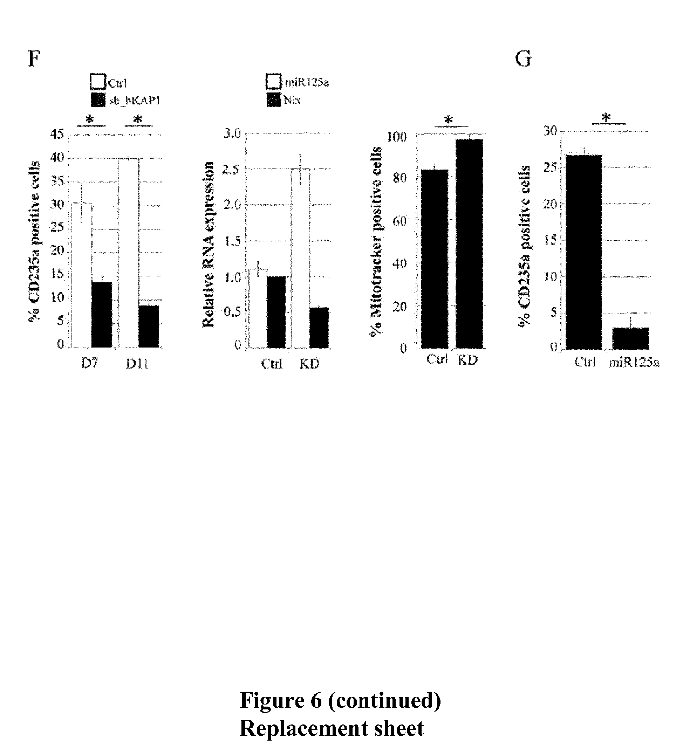

**Figure 6** **shows that KAP1 controls mitophagy during human erythropoiesis.** HEL transduced with scramble- or Kap1-specific-shRNA-expressing lentiviral vectors and induced or not to differentiate were evaluated for Kap1 mRNA expression (A), and benzidine (B) (n=3, counting 100 cells for each condition) or Mitotracker (C) (n=3, *p <0.05) staining. (D) hsa-miR-125a-5p (miR125a) and Nix expression measured respectively by NanoString nCounter direct RNA quantification and RNA sequencing in HEL cells transduced with empty or KAP1 knockdown vectors. (E) Nix expression was measured by RT-QPCR in Ctrl (setting normalized value at 1) or hsa-miR-125a-5p-overexpressing HEL cells (miR125a), measuring their mitochondrial content by Mitotracker staining (n=4, *p<0.05).(F) Human cord blood CD34+ cells transduced with GFP-encoding empty or Kap1-knockdown lentiviral vectors were induced two days later to undergo erythroid differentiation and the percentage of cells expressing the erythroid-specific surface marker CD235a was assessed by FACS after seven (D7) and eleven (D11) days. At D7, CD235a+eGFP+ cells were further sorted and analyzed by RT-QPCR for Nix and hsa-miR125a expression, and for mitochondrial content by Mitotracker staining (n=3, *p<0.05). (G) hsa-miR-125a overexpression blocks the erythroid differentiation of human CD34+ cells. Percentage of CD235a-expressing cells was assessed by FACS 7 days after inducing the differentiation of CD34+ cells transduced with empty or unrelated-miRNA- (Ctrl) or hsa-miR-125a-5p-overexpressing lentiviral vectors (n=3, *p<0.05).

### Detailed Description of the invention

### Definitions

The terms "Micro-RNA", "miRNA", and "miR" designate herewith a class of 17-25 nucleotides non-coding single stranded RNA molecules that regulate the expression of target genes by either translational inhibition or mRNAs degradation. Animal miRNAs typically exhibit only partial complementarity to their mRNA targets. A 'seed region' of about 6-8 nucleotides in length at the 5' end of an animal miRNA is thought to be an important determinant of target specificity. In animals, miRNAs are generated from primary transcripts (pri-miRNAs) generally transcribed from polymerase-II (Pol-II) promoters and processed in the nucleus by Drosha enzyme to about 60-70 nucleotides stem-loop (sl) molecules with two-nucleotide 3' overhangs (pre-miRNA). Pre-miRNAs are shuttled by exportin 5 to the cytosol where Dicer enzyme releases an about 21-24 double-stranded RNA from the stem. This is loaded into the RNA-induced silencing complex (RISC), which generally selects one of the two strands as the guide strand (mature miRNA), according to thermodynamic properties. RISC targets mRNA with complementary sequence to the miRNA and downregulates their expression decreasing transcript translation and stability by a variety of molecular mechanisms. The term "miRNA" is used herewith in a broad sense, including the mature miRNA, the pre-miRNA, or a variant thereof, as well as any small interfering RNA (siRNA) perfectly complementary to the transcript of the gene targeted by the miRNA.

The term "variant of a miRNA" as referred to herein, means a nucleic acid (either RNA or DNA) substantially homologous to the original miRNA sequence, but which has at least one nucleotide different from that of the original sequence because of one or more deletions, insertions or substitutions. Substantially homologous means a variant nucleic acid sequence that is at least 80%, at least 85%, at least 90%, at least 95%, at least 96%, at least 97%, at least 98% or at least 99% identical to the original nucleic acid sequence. The variant of a miRNA as defined herewith is capable of regulating the expression of said mi-RNA's target gene.

The term "variant" applied to a KRAB-ZFP protein, as referred to herein, means a polypeptide substantially homologous to the original KRAB-ZFP amino acid sequence, but which has at least one amino acid different from that of the original sequence because of one or more deletions, insertions or substitutions. Substantially homologous means a variant amino acid sequence that is at least 80%, at least 85%, at least 90%, at least 95%, at least 96%, at least 97%, at least 98% or at least 99% identical to the original amino acid sequence. The variant of a KRAB-ZFP protein as defined herewith is capable of regulating the expression of the mi-RNA target of said KRAB-ZFP, i.e. it is able to repress the expression of the target mi-RNA. A variant of a KRAB-ZFP protein can have the same DNA-binding domain as the original KRAB-ZFP protein, but a different transcription repressing module instead of KRAB, for instance a SCAN domain (Itokawa et al, 2009, Biochem Biophys Res Commun, 388(4): 689-94).

The terms "Percentage of identity", "% identity", or the like, refer to the level of identity between two nucleic acid sequences or two amino acid sequences, depending on whether nucleic acid sequences or amino acid sequences are compared. The percentage of identity of two sequences can be determined by visual inspection and/or mathematical calculation, or more easily by comparing sequence information using known computer program used for sequence comparison such as Clustal package version 1.83.

The expression "micro-RNA targeting a mitophagy-associated gene" designates herewith a micro-RNA that specifically regulates the expression of genes which, at least partially, play a role in mitophagy.

"Mitophagy" corresponds to the selective autophagy of mitochondria. This is a process by which a cell regulates its number of mitochondria depending on its energetic needs.

This process also constitutes an important mitochondrial quality control mechanism that eliminates damaged mitochondria. Mitophagy can be analysed by detection of mitochondria by electron microscopy or fluorescence microscopy, as well as by determination of mitochondrial mass or Western blot analysis for mitochondrial preoteins as disclosed in Ding and Yin (Biol. Chem. 2012, 393, 547*).*

"Mitophagy-associated genes" designates herewith genes which, at least partially, play a role in mitophagy. Examples of mitophagy-associated genes include Bnip3L in mouse, equivalent to Nix in humans.

"Mitochondria-related diseases" as defined herewith designate diseases mediated or influenced, at least in part, by mitochondria damage and/or a deregulated mitophagy process or an unsuitably high or low mitochondria content in certain types of cells. In mitochondria-related diseases, mitochondria damages can affect (i) red blood cells as for instance in anemia, polycythemia and erythroleukemia, (ii) adipocytes or cardiomyocytes as in lipid-related diseases including obesity and heart diseases, (iii) neuronal cells as in neurodegenerative diseases including Parkinson's disease, Alzheimer's disease and Huntington disease, or (iv) any other cell type as in diabetes, ischemia or drug-induced tissue injury, mitochondria-mediated tissue injury including hepatic necrosis, liver damage and steatosis, age-related diseases, aging, tumors and cancers, and inflammation.

The terms "short hairpin RNA", "small hairpin RNA" and "shRNA" designate herewith a sequence of RNA that makes a tight hairpin turn that can be used to silence target gene expression via RNA interference (RNAi). Expression of shRNA in cells is typically accomplished by delivery of plasmids or through viral or bacterial vectors.

As used herewith an "antagomir" designates a small synthetic RNA that is perfectly complementary to a specific miRNA target with either mispairing at the cleavage site of Ago2 or nucleotide modification to inhibit Ago2 cleavage. Usually, antagomirs have some chemical modifications, such as 2' methoxi groups and phosphothioates, to make them more resistant to degradation. Production of antagomirs may be achieved for instance as disclosed in Krutzfeldt et al (2005, Nature, 438, 685-689*)*.

An "inhibitor of a micro-RNA" as defined herewith designates any molecule, e.g. a polypeptide, a nucleic acid or a small chemical molecule, that is able to repress said micro-RNA expression or inhibit said micro-RNA function. For instance, the function of a micro-RNA can be inhibited by a molecule such as a nucleic acid sequence complementary to the micro-RNA's seed region.

A "sponge vector" designates herewith a vector, in particular a lentiviral vector, for overexpressing miRNA target sequences complementary to a miRNA seed region. The nucleic acid sequence comprised in the sponge vector does not need to be perfectly complementary to the miRNA seed region for inhibiting said micro-RNA function.

A "mutation" designates herewith a change in the nucleotide sequence of the genome of a subject compared to a control subject. As used herewith the term "mutation" includes ponctual mutation, substitution, insertion, deletion, and polymorphism within a nucleic acid sequence.

The term "subject" as used herein refers to mammals. For example, mammals contemplated by the present invention include human, primates, domesticated animals such as cattle, sheep, pigs, horses, laboratory rodents and the like.

The expression "biological sample" refers to a clinical sample for testing which is taken from a mammal such as saliva, blood, urine, and biopsy material.

The expression "control sample" refers to a negative control sample. A negative control sample includes a biological sample taken from a subject that is of the same or homologous species as the subject to be assayed for miRNA and/or KRAB-ZFP proteins and is known to have a normal biological state, e.g. without detectable defect in the mitophagy process and/or without detectable symptoms of a mitochondria-related disease. A negative control sample includes a sample taken from a control subject.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it for example based on familial history; (b) inhibiting the disease, i.e., arresting its development; or relieving the disease, i.e., causing regression of the disease and/or its symptoms or conditions such as improvement or remediation of damage.

In particular, treatment of anemia or erythroleukemia is accompanied by an increase in the number of mature red blood cells. Treatment of polycythemia is accompanied by a decrease in the number of mature red blood cells.

The term "efficacy" of a treatment or method according to the invention can be measured based on changes in the course of disease or condition in response to a use or a method according to the invention. For example, the efficacy of a treatment or method according to the invention can be measured by an increase in the number of mature red blood cells.

The term "effective amount" as used herein refers to an amount of a therapeutic agent that elicits a detectable change in the number of mitochondria within a given cell type in a subject that is being administered said therapeutic agent. In the case of mitochondria-related diseases affecting red blood cells, an effective amount of a therapeutic agent is the amount that elicits a regulation in the number of mature red blood cells in the blood or that promotes the maturation of erythrocytes in an *in vitro* red blood cells manufacturing protocol.

### Agents and Compositions

The invention provides agents and compositions capable of modulating mitophagy and, thus, are useful as medicament and, in particular, in the prevention or treatment of mitochondria-related diseases.

In a first embodiment, the invention provides an agent or composition comprising at least one of: (i) a micro-RNA targeting a mitophagy-associated gene or a variant thereof, (ii) a vector comprising a nucleic acid encoding said micro-RNA or variant thereof, (iii) an inducer of said micro-RNA, and (iv) an inhibitor of said micro-RNA, for use as a medicament.

The invention also provides the use of the agent or composition comprising at least one of: (i) a micro-RNA targeting a mitophagy-associated gene or a variant thereof, (ii) a vector comprising a nucleic acid encoding said micro-RNA or variant thereof, (iii) an inducer of said micro-RNA, and (iv) an inhibitor of said micro-RNA, in the manufacture of a medicament.

In a particular aspect of the invention, said micro-RNA is selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), mmu-miR-669f (SEQ ID NO: 12), or a variant thereof. In a more particular aspect, said micro-RNA is hsa-miR-125a-5p (SEQ ID NO: 1), mmu-miR-351 (SEQ ID NO: 3), or a variant thereof.

In another aspect, the agent or composition according to the invention comprises an inducer of a micro-RNA targeting a mitophagy-associated gene, which is a shRNA that silences a nucleic acid encoding a Kruppel-associated box zinc finger protein (KRAB-ZFP). More particularly, said inducer is a shRNA that silences a nucleic acid encoding a KRAB-ZFP selected among: human ZNF205 (SEQ ID NO: 13), human ZNF689 (SEQ ID NO: 14), mouse ZFP13 (SEQ ID NO: 15), and mouse ZFP689 (SEQ ID NO: 16).

In a still other aspect, the agent or composition according to the invention comprises an inhibitor of a micro-RNA targeting a mitophagy-associated gene, wherein said inhibitor is:
(i) a Kruppel-associated box zinc finger protein (KRAB-ZFP) repressing said micro-RNA expression, in particular a KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), or a variant thereof,
(ii) a vector over-expressing a KRAB-ZFP under (i) or variant thereof,
(iii) an antagomir repressing said micro-RNA expression, or
(iv) a sponge vector inhibiting said micro-RNA function.

A sponge vector useful in the present invention is a vector, in particular a lentiviral vector, for overexpressing miRNA target sequences complementary to a miRNA seed region. The nucleic acid sequence comprised in the sponge vector does not need to be perfectly complementary to the miRNA seed region for inhibiting said micro-RNA function. Thus, miRNA target sequences comprised in the sponge vector can be complementary to the seed region of the miRNA according to the invention except for a few (e.g. about 1, 2, 3) non complementary nucleotides. The exact number of non-complementary nucleotides are determined empirically for each targeted miRNA, by the skilled person. The miRNA seed region corresponds to about 6-8 nucleotides in length at the 5' end of a miRNA that is thought to be an important determinant of target specificity. The seed region of hsa-miR-125a-5p (SEQ ID NO: 1) corresponds to nucleotides 2 to 8 of SEQ ID NO: 1, the seed region of hsa-miR-125b-5p (SEQ ID NO: 2) corresponds to nucleotides 2 to 8 of SEQ ID NO: 2, the seed region of mmu-miR-351 (SEQ ID NO: 3) corresponds to nucleotides 2 to 8 of SEQ ID NO: 3, the seed region of mmu-miR-125a-5p (SEQ ID NO: 4) corresponds to nucleotides 2 to 8 of SEQ ID NO: 4, the seed region of mmu-miR-135a* (SEQ ID NO: 5) corresponds to nucleotides 2 to 8 of SEQ ID NO: 5, the seed region of mmu-miR-149 (SEQ ID NO: 6) corresponds to nucleotides 2 to 8 of SEQ ID NO: 6, the seed region of mmu-miR-338-3p (SEQ ID NO: 7) corresponds to nucleotides 2 to 8 of SEQ ID NO: 7, the seed region of mmu-miR-683 (SEQ ID NO: 8) corresponds to nucleotides 2 to 8 of SEQ ID NO: 8, the seed region of mmu-miR-574-3p (SEQ ID NO: 9) corresponds to nucleotides 2 to 8 of SEQ ID NO: 9, the seed region of mmu-miR-467d* (SEQ ID NO: 10) corresponds to nucleotides 2 to 8 of SEQ ID NO: 10, the seed region of mmu-miR-467a* (SEQ ID NO: 11) corresponds to nucleotides 2 to 8 of SEQ ID NO: 11, the seed region of mmu-miR-669f (SEQ ID NO: 12) corresponds to nucleotides 2 to 8 of SEQ ID NO: 12.

In particular, said inhibitor is an antagomir repressing the expression of hsa-miR-125a-5p (SEQ ID NO: 1) or of mmu-miR-351 (SEQ ID NO: 3) or a sponge vector inhibiting the function of hsa-miR-125a-5p (SEQ ID NO: 1) or of mmu-miR-351 (SEQ ID NO: 3).

In another embodiment, the invention relates to a pharmaceutical composition comprising: (i) a micro-RNA targeting a mitophagy-associated gene or a variant thereof, (ii) a vector comprising a nucleic acid encoding said micro-RNA or a variant thereof, (iii) an inducer of said micro-RNA, or (iv) an inhibitor of said micro-RNA; and a pharmaceutically acceptable carrier.

The invention provides pharmaceutical or therapeutic agents as compositions and methods for treating a subject, preferably a mammalian subject, and most preferably a human patient who is suffering from a medical disorder, and in particular a disorder mediated by impairments in mitophagy process, in particular anemia, polycythemia and erythroleukemia.

Compositions or formulations according to the invention may be administered as a pharmaceutical formulation which can contain one or more agents according to the invention in any form described herein.

The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions of this invention may be liquid formulations including, but not limited to, aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.

Compositions of this invention may also be formulated as a depot preparation, which may be administered by implantation or by intramuscular injection.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate.

Tablets may be coated according to methods well known in the art.

The compounds of this invention can also be administered in sustained release forms or from sustained release drug delivery systems.

According to a particular embodiment, compositions according to the invention are for intravenous use.

In another particular aspect, the compositions according to the invention are adapted for delivery by repeated administration.

Further materials as well as formulation processing techniques and the like are set out in *Part 5 of* Remington's "The Science and Practice of Pharmacy", 22nd Edition, 2012, University of the Sciences in Philadelphia, Lippincott Williams & Wilkins*,* which is incorporated herein by reference.

When the agents and compositions according to the invention comprise nucleic acids encoding micro-RNAs or KRAB-ZFPs as described herewith, those can be prepared by incorporating the nucleic acid as an active ingredient together with a base which is commonly used for an agent for gene therapy. When nucleic acids are incorporated into a virus vector, virus particles containing recombinant vectors are prepared, and the resultant are incorporated with a base which is commonly used for an agent for gene therapy.

As a base used for incorporating the nucleic acid as an active ingredient, a base that is commonly used for an injection can be used. Examples include distilled water, a salt solution of sodium chloride or a mixture of sodium chloride and an inorganic salt, a solution of mannitol, lactose, dextran, or glucose, an amino acid solution of glycine or arginine, an organic acid solution, and a mixed solution of a salt solution and a glucose solution. Alternatively, an adjuvant, such as a regulator of osmotic pressure, a pH adjuster, vegetable oil, or a surfactant, may be added to the base in accordance with a technique known in the art to prepare an injection in the form of a solution, suspension, or dispersion. Such injection can be prepared in the form of a preparation to-be-dissolved before use via operations, such as pulverization or lyophilization.

### Nucleic acids, vectors, and methods for making the agents and compositions according to the invention

Nucleic acids useful for the invention are those encoding at least one of: (i) a micro-RNA targeting a mitophagy-associated gene or a variant thereof, (ii) an inducer of said micro-RNA, and (iii) an inhibitor of said micro-RNA, as described herewith.

In a particular aspect, the nucleic acids useful for the invention encode micro-RNAs selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a*

(SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d*

(SEQ ID NO: 10), mmu-miR-467a* (SEQ ID NO: 11), mmu-miR-669f (SEQ ID NO: 12), or a variant thereof.

In a more particular aspect, a nucleic acid useful for the invention encodes a micro-RNA consisting of hsa-miR-125a-5p (SEQ ID NO: 1), mmu-miR-351 (SEQ ID NO: 3), or a variant thereof.

In another aspect, a nucleic acid useful for the invention encodes a shRNA that silences a nucleic acid encoding a Kruppel-associated box zinc finger protein (KRAB-ZFP).

More particularly, a nucleic acid useful for the invention is a shRNA that silences a nucleic acid encoding a KRAB-ZFP selected among: human ZNF205 (SEQ ID NO: 13), human ZNF689 (SEQ ID NO: 14), mouse ZFP13 (SEQ ID NO: 15), and mouse ZFP689 (SEQ ID NO: 16).

In a still other aspect, a nucleic acid useful for the invention encodes an inhibitor of a micro-RNA targeting a mitophagy-associated gene.

In a particular aspect, said nucleic acid encodes a Kruppel-associated box zinc finger protein (KRAB-ZFP) selected among: human ZNF205 (SEQ ID NO: 13), human ZNF689 (SEQ ID NO: 14), mouse ZFP13 (SEQ ID NO: 15), mouse ZFP689 (SEQ ID NO: 16), or a variant thereof.

In a still further particular aspect, said nucleic acid encodes a Kruppel-associated box zinc finger protein (KRAB-ZFP) selected among: human ZNF205 (SEQ ID NO: 13) human ZNF689 (SEQ ID NO: 14), or a variant thereof.

In a particular aspect, said nucleic acid encoding said KRAB-ZFP is selected among: human nucleic acid ZNF205 (SEQ ID NO: 17), human nucleic acid ZNF689 (SEQ ID NO: 18), mouse nucleic acid ZFP13 (SEQ ID NO: 19), and mouse nucleic acid ZFP689 (SEQ ID NO: 20), or variant thereof having at least 80%, at least 85%, at least 90%, at least 95%, at least 96%, at least 97%, at least 98%, or at least 99% identity thereof.

In a still further particular aspect, said nucleic acid encoding said KRAB-ZFP is selected among: human nucleic acid ZNF205 (SEQ ID NO: 17), human nucleic acid ZNF689 (SEQ ID NO: 18), or a variant thereof having at least 80%, at least 85%, at least 90%, at least 95%, at least 96%, at least 97%, at least 98%, or at least 99% identity thereof.

In a further particular aspect, said nucleic acid encodes an antagomir repressing a micro-RNA according to the invention.

Another aspect of the invention provides vectors comprising the nucleic acids described above. In particular, it is provided cloning vectors, expression vectors, sponge vectors, as described herewith.

MiRNAs targeting a microphagy-associated gene according to the invention or their primary transcripts (pre-miRNA) can be cloned in a retroviral vector, preferentially in a lentiviral vector, or an adenovirus vector, to be subsequently delivered to the cells, according to standard techniques in the art such as the one disclosed in Amendola et al (2009, Molecular Therapy, 17(6), 1039-1052*)* using a lentiviral vector comprising mi-RNA.

For making over-expressing KRAB-ZFPs vectors according to the invention, the nucleic acid encoding said KRAB-ZFP is cloned in viral vectors such as adeno-associated viruses (AAVs), adenoviruses, and lentivirus, according to standard techniques in the art. For obtaining an over-expression of said KRAB-ZFP, the nucleic acid encoding said KRAB-ZFP is placed under the control of an inducible system such as the Tet-ON system which allows the expression of the gene only in presence of an inducer, the antibiotic tetracycline or one of its derivatives (e.g. doxycycline).

Making an antagomir according to the invention can be achieved by chemically synthesis as described in Krutzfeldt et al (2005, Nature, 438, 685-689*)*.

For making a sponge vector, in particular a lentiviral vector, for inhibiting miRNA function according to the invention, a miRNA target sequences complementary to a miRNA seed region of the miRNA according to the invention is cloned in a lentiviral vector as described for instance in Gentner et al (2009, Nature Methods, 6(1), 63-66*).*

It is understood that the vectors mentioned above comprise at least one expression control element operably linked to a nucleic acid sequence encoding at least one of the micro-RNA according to the invention.

### Uses and methods according to the invention in therapy

A still other aspect of the invention provides a method of modulating mitophagy in a subject.

In one aspect, the invention provides a method of reducing mitophagy in a subject comprising administering an agent or composition according to the invention, to said subj ect.

In a particular embodiment, the method of reducing mitophagy according to the invention comprises administering to said subject any one of:
(i) a micro-RNA targeting a mitophagy-associated gene or a variant thereof,
(ii) a vector comprising a nucleic acid encoding said micro-RNA or variant thereof,
(iii) an inducer of said micro-RNA,
   wherein said micro-RNA is selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), and mmu-miR-669f (SEQ ID NO: 12); or said inducer is a shRNA that silences a nucleic acid encoding a KRAB-ZFP selected among: human ZNF205 (SEQ ID NO: 13), human ZNF689 (SEQ ID NO: 14), mouse ZFP13 (SEQ ID NO: 15), and mouse ZFP689 (SEQ ID NO: 16).

In another aspect, the invention provides a method of increasing mitophagy in a subject comprising administering an agent or composition according to the invention, to said subj ect.

In a particular embodiment, the method of increasing mitophagy according to the invention comprises administering to said subject an inhibitor of a micro-RNA targeting a mitophagy-associated gene, selected among:
(i) a Kruppel-associated box zinc finger protein (KRAB-ZFP) selected among: human ZNF205 (SEQ ID NO: 13), human ZNF689 (SEQ ID NO: 14), mouse ZFP13 (SEQ ID NO: 15), mouse ZFP689 (SEQ ID NO: 16), or a variant thereof,
(ii) a vector over-expressing a KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), or a variant thereof, (iii) an antagomir repressing said micro-RNA expression, or (iv) a sponge vector inhibiting said micro-RNA function,
wherein said micro-RNA is selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), and mmu-miR-669f (SEQ ID NO: 12).

In a particular embodiment of the invention, the vector under (ii) comprises a nucleic acid selected among: human nucleic acid ZNF205 (SEQ ID NO: 17), human nucleic acid ZNF689 (SEQ ID NO: 18), mouse nucleic acid ZFP13 (SEQ ID NO: 19), and mouse nucleic acid ZFP689 (SEQ ID NO: 20), or a variant thereof having at least 80%, at least 85%, at least 90%, at least 95%, at least 96%, at least 97%, at least 98%, or at least 99% identity thereof.

In another object, the invention provides a method of treating, preventing, or attenuating a mitochondria-related disease in a subject, comprising administering in a subject in need thereof an effective amount of an agent or composition comprising at least one of: (i) a micro-RNA targeting a mitophagy-associated gene or a variant thereof, (ii) a vector comprising a nucleic acid encoding said micro-RNA or variant thereof, (iii) an inducer of said micro-RNA, and (iv) an inhibitor of said micro-RNA, as described herewith.

In a still other object, the invention provides the use of at least one of: (i) a micro-RNA targeting a mitophagy-associated gene or a variant thereof, (ii) a vector comprising a nucleic acid encoding said micro-RNA or variant thereof, (iii) an inducer of said micro-RNA, and (iv) an inhibitor of said micro-RNA, as described herewith, in the manufacture of a medicament for treating, preventing, or attenuating a mitochondria-related disease.

In a particular embodiment of the method of treating, preventing or attenuating a mitochondria-related disease and of the use in the manufacture of a medicament for treating, preventing, or attenuating a mitochondria-related disease, according to the invention, said mitochondria-related disease is selected among red blood cells-related diseases including anemia, polycythemia and erythroleukemia, lipid-related diseases including obesity and heart diseases, diabetes, neurodegenerative diseases including Parkinson's disease, Alzheimer's disease and Huntington disease, ischemia or drug-induced tissue injury, mitochondria-mediated tissue injury including hepatic necrosis, liver damage and steatosis, age-related diseases, aging, tumors and cancers and inflammation.

In a still further particular embodiment, the method and uses according to the invention are for treating, preventing or attenuating a mitochondria-related disease affecting red blood cells such as anemia, polycythemia and erythroleukemia.

In a still other aspect, there is provided herein, a method of treating a mitochondria-related disease in a subject suffering therefrom in which said at least one micro-RNA is down-regulated or up-regulated in said subject's cells relative to control cells as determined by the *ex-vivo* method of prognosis and/or diagnosis of a mitochondria-related disease according to the invention described below, comprising:
a) when said at least one micro-RNA is down-regulated in the subject's cells, administering to the subject an effective amount of at least one isolated micro-RNA, such that mitophagy decreases in the subject's cells; or
b) when said at least one micro-RNA is up-regulated in the subject's cells, administering to the subject an effective amount of at least one compound inhibiting expression of said at least one micro-RNA, such that mitophagy increases in the subject's cells.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Mode of administration

Compositions of this invention may be administered in any manner including intravenous injection, intra-arterial, intraperitoneal injection, subcutaneous injection, intramuscular, intra-thecal, oral route, cutaneous application, direct tissue perfusion during surgery or combinations thereof.

The compositions of this invention may also be administered in the form of an implant, which allows slow release of the compositions as well as a slow controlled i.v. infusion. Delivery methods for the composition of this invention include known delivery methods for anti-cancer drugs such as intra-venal peripheral injection, intra-tumoral injection or any type of intracranial delivery such as convection enhanced delivery (CED) (Bobo et al., 1994, PNAS, 91 (6), 2076-2080*;* Lino et al., 2009, Curr. Opin. Cell Biol., 21, 311-316*).*

When the agents and compositions according to the invention comprise nucleic acids encoding micro-RNAs or KRAB-ZFPs, or vectors, as described herewith, as active ingredients, those can be administered via, for example, injection of naked DNA, electroporation, the gene gun, sonoporation, magnetofection, or a method using oligonucleotides, lipoplexes, dendrimers, and inorganic nanoparticles. Those agents can also be administered to the cells or tissues of a subject after having been incorporated into DNA or RNA viruses, including retrovirus, adenovirus, lentivirus, herpes simplex virus, vaccinia, pox virus, adenovirus, and adeno-associated virus.

### Combination

According to the invention, the agents and compositions according to the invention, and pharmaceutical formulations thereof can be administered alone or in combination with a co-agent useful in the treatment of a mitochondria-related disease.

The invention encompasses the administration of an agent or composition according to the invention and pharmaceutical formulations thereof, wherein said agent or composition is administered to an individual prior to, simultaneously or sequentially with other therapeutic regimens or co-agents useful in the treatment of a mitochondria related disease, in particular those affecting red blood cells, (e.g. multiple drug regimens), in a therapeutically effective amount.

An agent or composition according to the invention, or the pharmaceutical formulation thereof, that is administered simultaneously with said co-agents can be administered in the same or different composition(s) and by the same or different route(s) of administration.

According to one embodiment, is provided a pharmaceutical formulation comprising a agent or composition according to the invention, combined with at least one co-agent useful in the treatment of a mitochondria-related disease, preferably those affecting red blood cells, in particular anemia, polycythemia and erythroleukemia, and at least one pharmaceutically acceptable carrier.

### Uses and methods according to the invention in diagnostics

In another aspect of the invention is provided an *ex-vivo* method of prognosis and/or diagnosis of a mitochondria-related disease in a subject comprising determining, in a biological sample of said subject, the level of at least one of: (i) a micro-RNA targeting a mitophagy-associated gene, (ii) a KRAB-ZFP, (iii) a mitophagy-associated gene.

In a particular aspect of the invention is provided an *ex-vivo* method of prognosis and/or diagnosis of a mitochondria-related disease in a subject comprising determining the level of at least one of: (i) a micro-RNA targeting a mitophagy-associated gene, (ii) a KRAB-ZFP selected among: human ZNF205 (SEQ ID NO: 13), human ZNF689 (SEQ ID NO: 14), mouse ZFP13 (SEQ ID NO: 15), and mouse ZFP689 (SEQ ID NO: 16), (iii) a mitophagy-associated gene; in a biological sample from said subject.

In a particular embodiment, the *ex-vivo* method of prognosis and/or diagnosis of a mitochondria-related disease according to the invention comprises:
a) Providing a biological sample from a subject;
b) Determining the level of at least one micro-RNA selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), mmu-miR-669f (SEQ ID NO: 12), in said sample;
c) Comparing the level of step b) with the level of a corresponding micro-RNA in a control sample:
wherein an alteration of the level of said micro-RNA determined in step b) compared to the control sample is indicative of the subject being at risk for developing, or having, a mitochondria-related disease.

In a specific embodiment, the *ex-vivo* method of prognosis and/or diagnosis of a mitochondria-related disease according to the invention comprises:
a) Providing a biological sample from a subject;
b) Reverse transcribing RNA from said biological sample to provide a set of target oligodeoxynucleotides;
c) Hybridizing the target oligodeoxynucleotides to a microarray comprising at least one micro-RNA-specific probe oligonucleotides to provide a hybridization profile for said biological sample, wherein said micro-RNAs are selected among hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), and mmu-miR-669f (SEQ ID NO: 12); and
d) Comparing said biological sample hybridization profile to a hybridization profile generated from a control sample,
wherein an alteration in the signal of at least one of said micro-RNAs is indicative of the subject being at risk for developing, or having, a mitochondria-related disease.

In the *ex-vivo* method of prognosis and/or diagnosis of a mitochondria-related disease according to the invention, the alteration of the level of said micro-RNA in the biological sample of a subject may correspond to an up-regulation or a down-regulation of said micro-RNA in the subject's cells.

In an alternative embodiment, the *ex-vivo* method of prognosis and/or diagnosis of a mitochondria-related disease according to the invention comprises:
a) Providing a biological sample from said subject;
b) Determining the level of at least one KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), in said sample;
c) Comparing the level of step b) with the level of a corresponding KRAB-ZFP in a control sample:
wherein an alteration of the level of said KRAB-ZFP determined in step b) compared to the control sample is indicative of the subject being at risk for developing, or having, a mitochondria-related disease.

In the methods mentioned herewith, the level of micro-RNA and the level of KRAB-ZFP transcripts can be determined, for instance, by the DNA array method, Northern blotting, RT-PCR, real-time PCR, RT-LAMP, or in situ hybridization.

Further, in the methods mentioned herewith, the level of KRAB-ZFP can be determined, for instance, by Western blotting, immunodetection, or by determination of the level of KRAB-ZFP transcript (i.e. RNA).

In a still further aspect of the invention is provided an *ex-vivo* method of prognosis and/or diagnosis of a mitochondria-related disease in a subject comprising determining whether a biological sample from said subject comprises a nucleic acid which comprises a mutation in at least one of: (i) a micro-RNA targeting a mitophagy-associated gene, (ii) a KRAB-ZFP, (iii) the nucleic acid sequence of the DNA target of a KRAB-ZFP, (iv) a mitophagy-associated gene.

In another aspect of the invention is provided an *ex-vivo* method of prognosis and/or diagnosis of a mitochondria-related disease in a subject comprising determining, in a biological sample from said subject, the presence of a nucleic acid comprising a mutation in at least one of: (i) a micro-RNA targeting a mitophagy-associated gene, (ii) a gene encoding a KRAB-ZFP selected among: human ZNF205 (SEQ ID NO: 13), human ZNF689 (SEQ ID NO: 14), mouse ZFP13 (SEQ ID NO: 15), and mouse ZFP689 (SEQ ID NO: 16), (iii) a nucleic acid sequence selected among: human nucleic acid ZNF205 (SEQ ID NO: 17), human nucleic acid ZNF689 (SEQ ID NO: 18), mouse nucleic acid ZFP13 (SEQ ID NO: 19), mouse nucleic acid ZFP689 (SEQ ID NO: 20), (iv) the nucleic acid sequence of the DNA target of at least one KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), (v) a mitophagy-associated gene.

In a further embodiment, the *ex-vivo* method of prognosis and/or diagnosis of a mitochondria-related disease according to the invention comprises:
a) Providing a biological sample from a subject;
b) Determining whether said biological sample comprises a nucleic acid which comprises a mutation:
   (i) in a nucleic acid sequence encoding at least one micro-RNA selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), mmu-miR-669f (SEQ ID NO: 12),
   (ii) in a nucleic acid sequence encoding at least one KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), and/or
   (iii) in a nucleic acid sequence selected among: human nucleic acid ZNF205 (SEQ ID NO: 17), human nucleic acid ZNF689 (SEQ ID NO: 18), mouse nucleic acid ZFP13 (SEQ ID NO: 19), mouse nucleic acid ZFP689 (SEQ ID NO: 20),
   (iv) in the nucleic acid sequence of the DNA target of at least one KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16),
   (v) in a mitophagy-associated gene,
whereby a mutation in a nucleic acid sequence encoding at least one of said micro-RNA and/or KRAB-ZFP and/or in the nucleic acid sequence of the DNA target of said KRAB-ZFP is indicative of a mitochondria-related disease in said subject.

Methods for detecting mutations are well known to the skilled person and include direct sequencing of PCR products, methods which rely on the differences in electrophoretic mobilities of wild-type and mutant nucleic acids such as those based on denaturing gradient gel electrophoresis, temperature gradient gel electrophoresis, single-strand conformation polymorphism, heteroduplex analysis, methods which rely on cleavage of heteroduplices such as those based on RNase A cleavage, chemical cleavage, enzyme mismatch cleavage, cleavase fragment length polymorphism, and methods based on mutation detection by mismatch binding proteins, as well as methods based on the use of allele-specific oligonucleotide hybridization on DNA chips.

In the *ex-vivo* method of prognosis and/or diagnosis of a mitochondria-related disease according to the invention, the biological sample can be selected among blood, tissue biopsy, urine, saliva.

### Methods according to the invention for in vitro production of red blood cells

In another aspect, the invention provides a method for *in vitro* production of red blood cells comprising a step of inducing mitophagy in cells in culture by contacting said cells with an agent or composition comprising at least one inhibitor of a micro-RNA targeting a mitophagy-associated gene or a variant thereof.

In one embodiment, the method for *in vitro* production of red blood cells according to the invention is as defined above, wherein said inhibitor is (i) a Kruppel-associated box zinc finger protein (KRAB-ZFP) selected among: human ZNF205 (SEQ ID NO: 13), human ZNF689 (SEQ ID NO: 14), mouse ZFP13 (SEQ ID NO: 15), mouse ZFP689 (SEQ ID NO: 16), and a variant thereof, (ii) a vector over-expressing a KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), and a variant thereof, (iii) an antagomir repressing said micro-RNA expression, or (iv) a sponge vector inhibiting said micro-RNA function, and wherein said micro-RNA is selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), and mmu-miR-669f (SEQ ID NO: 12). The cells used in the method for *in vitro* production of red blood cells according to the invention include embryonic stem cells, induced pluripotent stem cells, multipotent precursor cells, hematopoietic stem cells, erythroid progenitor cells, which are all able to differentiate into red blood cells in specific culture conditions for instance as described in Nakamura Y, 2008 (Biotechnol Genetic Engineering Reviews, 25, 187-202*).*

Multipotent precursor cells can be isolated from bone marrow, umbilical cord blood or blood after cytokine-induced mobilization of bone marrow cells, hematopoietic stem cells can be isolated from bone marrow, umbilical cord blood of from blood after cytokine-induced mobilization of bone marrow cells, erythroid progenitor cells can be isolated from bone marrow, cord blood or blood after cytokine-induced mobilization of bone marrow cells or from erythroid progenitor cell lines, for instance. Induced pluripotent stem cells can be produced by transcription factor-induced reprogramming of any somatic cell as described in Maherali and Hochedlinger, 2008, Cell Stem Cell, 3(6):595-605 *;* Park et al, 2008, Nat Protoc, 3(7):1180-6 *;* Takahashi et al, 2007, Nat Protoc, 2(12):3081-9 *;* Ohnuki et al, 2009, Curr Protoc Stem Cell Biol. Chapter 4: Unit 4A.2*.)*

Depending on the cells used, the method for *in vitro* production of red blood cells according to the invention can further comprise steps wherein said cells are submitted to successive passages in specific culture media, such as those described in Nakamura Y, 2008 (Biotechnol Genetic Engineering Reviews, 25, 187-202*),* Giarratana et al (2005, Nature biotechnology, 23, 69-74) or Miharada et al (2006, Nature biotechnology, 24, 1255-6).

According to the method of the invention, the induction of mitophagy can take place during any step of erythroblast maturation.

### Subjects

In an embodiment, subjects according to the invention are subjects suffering from a mitochondria-related disease, or a disease at least partially related to mitochondria damages or a deregulation in mitochondria number.

In another embodiment, subjects according to the invention are subjects suffering from a mitochondria-related disease selected from red blood cells-related diseases including anemia, polycythemia and erythroleukemia, lipid-related diseases including obesity and heart diseases, diabetes, neurodegenerative diseases including Parkinson's disease, Alzheimer's disease and Huntington disease, ischemia or drug-induced tissue injury, mitochondria-mediated tissue injury including hepatic necrosis, liver damage and steatosis, age-related diseases, aging, tumors and cancers and inflammation.

In another embodiment, subjects according to the invention are subjects suffering from red blood cells-related diseases, in particular anemia, polycythemia and erythroleukemia.

### Use and methods according to the invention for screening

In another aspect, the invention provides a method of identifying a compound capable of modulating mitophagy.

In a particular aspect of the invention, is provided a method of identifying a compound capable of modulating mitophagy, comprising:
a) providing a cell comprising a nucleic acid sequence encoding at least one micro-RNA selected from hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), mmu-miR-669f (SEQ ID NO: 12), or a variant thereof;
b) contacting a test compound with the cell under a);
c) determining the level of at least one of said micro-RNA in said cell, with and without test compound;
wherein a difference in the level of at least one of said micro-RNA is indicative of a compound capable of modulating mitophagy.

The method as described above, wherein an increase in the level of at least one of said micro-RNA in presence of said test compound is indicative of a compound capable of decreasing mitophagy, while a decrease in the level of at least one of said micro-RNA in presence of said test compound is indicative of a compound capable of increasing mitophagy.

In another particular aspect of the invention, is provided a method of identifying a compound capable of modulating mitophagy, comprising:
a) providing a cell comprising a nucleic acid sequence encoding at least one KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), or a variant thereof,
b) contacting a test compound with the cell under a);
c) determining the level of at least one of said KRAB-ZFP in said cell, with and without test compound;
wherein a difference in the level of at least one of said KRAB-ZFP is indicative of a compound capable of modulating mitophagy.

The method as described above, wherein a decrease in the level of at least one of said KRAB-ZFP in presence of said test compound is indicative of a compound capable of decreasing mitophagy, while an increase in the level of at least one of said KRAB-ZFP in presence of said test compound is indicative of a compound capable of increasing mitophagy.

### Kits

Another object of the invention is a kit for carrying out any one of the methods and uses according to the invention.

In a particular embodiment, the invention relates to a kit of parts for diagnosing/prognosing a mitochondria-related disease in a subject comprising a probe consisting of an isolated nucleic acid from 12 to 250 nucleotides in length and comprising at least 12 contiguous nucleotides at least 80%, at least 85%, at least 90%, at least 95%, or at least 99%, identical to any one of the sequences of: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), mmu-miR-669f (SEQ ID NO: 12), human ZNF205 (SEQ ID NO: 17), human ZNF689 (SEQ ID NO: 18), mouse ZFP13 (SEQ ID NO: 19), and mouse ZFP689 (SEQ ID NO: 20), or the complement thereof, reagents and instructions for use.

In another particular embodiment, the invention relates to a kit of parts for identifying a compound for its ability to modulate mitophagy; and/or prevent, treat, or attenuate a mitochondria-related disease in a subject, comprising the same constituents as described for the kit of parts for diagnosing/prognosing a mitochondria-related disease.

Reagents considered are those reagents particularly developed and designed for the detection and determination of the expression of micro-RNA and KRAB-ZFB according to the invention.

The kits of parts according to the invention may contain further hardware, such as pipettes, solutions such as buffers, blocking solutions and the like, filters, color tables and directions for use.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### Examples

The following abbreviations refer respectively to the definitions below:
**aa** (amino acid); **bp** (base pair), µ**l** (microliter), µ**M** (micromolar), **mM** (millimolar), **mg** (milligram), min (minute).

### Materials and methods

**Mouse experiments.** All mice were maintained in the EPFL animal facility under specific pathogen-free conditions and housed in individually ventilated cages. Animal procedures were performed according to protocols approved by the Swiss Bundesamt für Veterinärwesen Nb. 2082. A hematopoietic-restricted conditional Kap1 knockout mouse model was generated by first crossing homozygous Kap1flox animals (Tif1ßL3/L3; Cammas et al., 2000, Development 127, 2955) with heterozygous Mx-Cre mice, where the recombinase is expressed from the interferon (IFN)-inducible Mx1 promoter, which can be activated by administration of synthetic double-stranded RNA polyinosinic-polycytidylic acid (pIC) (Kuhn et al, 1995, Science 269, 1427). In order to facilitate the specific examination of Kap1-deleted cells, these mice were further crossed with the stopfloxYFP strain, in which the fluorescent marker YFP is produced following Cre-mediated excision owing to the presence of a loxP-flanked stop sequence between its promoter and coding sequence (Srinivas et al., 2001, BMC Dev Bio 1, 4). Finally, to restrict Kap1 excision to the hematopoietic system, bone marrow chimeric mice were generated by transplanting total bone marrow cells from Kap-flox/stopfloxYFP CD45.2 male mice, with or without the Mx-Cre transgene, into lethally irradiated CD45.1 female animals. Kap1 deletion was induced by pIC injection 6 to 8 weeks after transplantation, and the analyses began at least 2 weeks later, so as to avoid effects linked to pIC injection per se (Essers et al., IFNalpha activates dormant haematopoietic stem cells in vivo. Nature 458, 904).

C57BL/6 (CD45.2+) and B6.SJL-Ptprc(a)Pep(b)BoyCrl(Ly5.1) (CD45.1+) mice were purchased from Charles River. Generation and genotyping of mice with a floxable Kap1 allele (Kap1flox; Tif1ßL3/L3), the MxCre, and the Cre-reporter stopfloxYFP mouse strains have been described previously (Cammas et al., 2000, Development 127, 2955 *;* Kuhn et al, 1995, Science 269, 1427 *;* Srinivas et al., 2001, BMC Dev Biol 1, 4). Injection of pIC was done intraperitoneally with 5µg/g poly (I:C) (Invivogen). To generate chimaeras, transplantations were performed using total bone marrow cells from male mice CD45.2+ Kap-flox/stopfloxYFP with or without MxCre or in wild type CD45.1+ female mice. Competitive graft was performed with a mixture to a 50% ratio of CD45.2+ Kap-flox/stopfloxYFP/MxCre+ or not with CD45.1+ wild-type bone marrow. Bone marrow mixtures, representing a total amount of 5.10e6 cells, were injected intravenously (i.v.) into lethally irradiated recipient mice. LSK graft, transduction and FACS analysis were performed as previously described (Barde et al., 2011, Gene therapy 18, 1087).

**Vectors.** pLKO vectors were purchased from Sigma-Aldrich and the puromycin was replaced by eGFP.(pLKO-mKAP1, pLKO-hKAP1, pLKO-empty, pLKO-scramble. pLKO-ZFP13, pLKO-ZFP689). BNip3L cDNA or 3'UTR was cloned into the bidirectional vector obtained from L. Naldini *(*Amendola et al, 2005, Nat Biotechnol, 23(1):108-16*),* to create the Bid-Bnip3L and Bid-Bnip3L-3UTR respectively. Precursor of mmu-miR-351 and hsa-miR125a was cloned into the expression plasmid kindly provided by L. Naldini *(*Amendola et al, 2009, Mol Ther, 17(6): 1039-1052*)* with the tRFP in replacement of the orange fluorophore (pCCL-SFFV-IntronmiR351-tRFP, pCCL-SFFV-IntronmiR125a-tRFP and the pCCL-SFFV-Intron-TRFP as the empty control). The KAP1 pic identified by ChipSeq closed to the miR351 locus was cloned into the pRRL-hPGK-mSEAP (pRRL-picmiR351-hPGK-mSEAP). Sequences for all plasmids DNA are available at Addgene (http://www.addgene.org) for all the vectors described. Production and titration of lentiviral vectors was performed as previously described (Barde et al., 2011, Gene therapy 18, 1087).

**Cells.** MEL cells were kindly provided by Punam Malik. Cells at a concentration of 2 millions per ml were induced to differentiate during seven days in DMEM (Invitrogen) supplemented with 20% serum (Hyclone) and 5 mM HMBA (hexamethylene bisacetamide, Sigma-Aldrich).

HEL cells (Martin and Papayannopoulou, 1982, Science 216, 1233) were obtained from Giuliana Ferrari. They were induced to differentiate in presence of hemin (Sigma-Aldrich) at a final concentration of 50 micromolar during five days. Benzidine staining was performed using 1 ml 0.2% benzidine (Sigma-Aldrich) in acetic acid 3% solution, supplemented with 20 µl hydrogen peroxide (Sigma-Aldrich). Cells were incubated for 15 min on ice, prepared on slides by using a cytospin centrifuge and counted for the proportion of blue cells over total cells. All transductions were performed at a MOI of 100, based on titer obtained by FACS or QPCR quantification, at a cell concentration of 1 million per ml. To stain mitochondria, cells at a concentration of 4 millions per ml were labeled with 20 µ Mitotracker deep red or green (Molecular Probes) at 37 °C for 30 min, before analysis by flow cytometry.

CD34+ cells from human cord blood were obtained from the Lausanne University Hospital (Centre Hospitalier Universitaire Vaudois, CHUV, switzreland). Cells were layered on Ficoll gradient, purified with the CD34+ cell separation kit (Miltenyi Biotec) and frozen in StemSpan SFEM medium (Stem cells tehcnologies) supplemented with 10% serum (Hyclone), 10% dimethyl Sulfoxyde (Chemica).

After thawing CD34+ cells were expanded for one day in StemSpan SFEM medium supplied with cytokines (100 ng/ml of Flt-3 ligand, 100 ng/ml of SCF, 20 ng/ml of TPO, and 20 ng/ml of IL-6; StemCell Technologies Inc.) and 5% penicillin/streptomycin. Cells were maintained at a density of 2x105 cells/ml and transduced the day after thawing with lentiviral vector at a MOI of 100. Two days after the transduction, cells were pelleted and transferred into StemSpan SFEM medium with 5% penicillin/streptomycin, 20%serum, erythropoietin (1 U/ml), IL-3 (1 ng/ml), SCF (50 ng/ml), dexamethasone (1 µM), and -estradiol (1 µM). Cells were incubated at 37°C with 5% CO2 and maintained at a density of 1x105 cells/ml for 7 days, before resuspension and transfer at a concentration of 1x106 cells/ml into StemSpan SFEM medium with 10% serum and erythropoietin (2 U/ml) for 4 additional days. Erythroid differentiation was monitored by the expression of the cell surface marker CD235a.

**Transmission electron microscopy.** CD71+Ter119+ cells isolated from the bone marrow of control and Kap1 KO micd were fixed and embedded in Spurr's low viscosity resin. Sections were prepared and stained with uranyl acetate and lead citrate, and analyzed with a Hitachi H-7500 transmission electron microscope.

**RT-QPCR.** Total RNA was extracted using TRizol reagent (Invitrogen) according to the manufacturer's instructions. For real-time PCR, total RNA was reverse-transcribed with the Superscript VILO cDNA synthesis kit (Invitrogen). All primers used in this work were designed by Primer Express software (Applied Biosystems) or the GetPrime resource (Gubelmann et al., 2011, Database : the journal of biological databases and curation 2011, bar040); (http://updeplalsrv1.epfl.ch/getprime).

**Microarray.** RNA was extracted from CD71+Ter119+ cells FACS sorted from 4 Kap1 KO mice, YFP+ and Ctrl mice 7 weeks after pIC injection. Purification and preparation was done as previously described (Santoni de Sio et al., 2012, Blood 119, 4675) and hybridization was carried out on mouse WG 6 v2 expression arrays (Illumina). miRNA expression was determined on the same sample with the Illumina microRNA expression profiling assay.

**Chromatin immunoprecipitation (ChIP) sequencing.** 10 millions CD71+Ter119+ sorted cells from 8- to 12-wk-old Kap-flox/stopfloxYFP mouse bone marrow were crosslinked by 1% formaldehyde and sonicated, and chromatin was immunoprecipitated by using an affinity-purified rabbit polyclonal antibody raised against KAP1 aa 20-418, RBCC (Schultzet al, 2001, Genes Dev 15, 428). Detailed protocols for ChIP are described elsewhere (Santoni de Sio et al., 2012, FASEB J, Aug. 7*,* Meylan et al., 2011, BMC genomics 12, 378*).* Raw reads were mapped to the mouse genome (assembly mm9) using the bowtie short read aligner (Langmead et al, 2009, Genome biology 10, R25). Peaks were called using the Model-based Analysis of ChIP-Seq algorithm (Zhang et al., 2008, Genome biology 9, R137).

**RNA-Seq.** Total RNA was extract from HEL cells transduced with empty or Kap1 knockdown vectors using TRizol reagent (Invitrogen). RNAseq was performed on an Illumina HiSeq 2000 sequencer (single read 100 cycles assay). The library was generated from 250 ng total RNA using the TruSeq RNA Sample Preparation Kit v2. Raw reads were mapped to the human transcriptome (hg19) using the bowtie short read aligner (Langmead et al, 2009, Genome biology 10, R25) and counts were normalized to the transcript length and to the total number of reads (rpkm). Differentially expressed genes were characterized using the DESeq Bioconductor package (Anders and Huber, 2010, Genome biology 11, R106).

**Direct RNA quantification by NanoString nCounter.** KRAB-ZFP expression analysis was performed as previously described (Santoni de Sio et al., 2012, FASEB J, Aug. 7). Human miRNA expression was performed on same HEL cells RNA used for RNAseq, using the nCounter miRNA Expression Assay (HSA miRNA v2 assay kit) according to the manufacturer's recommendations (Nanostring) starting from 100 ng total RNA.

### Example 1: KAP1-deficient mice display a block in erythropoiesis due to defective mitophagy

Using a hematopoietic-restricted conditional Kap1 knockout mouse model it was determined that loss of KAP1 leads to a series of hematological abnormalities including thrombocytopenia, an increase in the percentage of monocytes and a drop in the number of circulating eosinophils (Figure 1D). Most prominent, however, was a picture of hypo-regenerative anemia, with the bone marrow of Kap1-deleted mice exhibiting a block of erythroblast maturation, with accumulation of transferrin receptor/CD71+ glycophorin-A-associated/Ter119- early erythroblasts and almost complete absence of mature CD71-Ter119+ cells (Figure 1B). Because the stage of CD71+Ter119+ erythroblasts, the most differentiated red cell precursors still detected in Kap1 KO animals, normally corresponds to the elimination of organelles, these cells were subjected to electron microscopy studies (Figure 1C). These revealed that KO erythroblasts contained markedly more mitochondria than their wild type (WT) counterparts, a point confirmed by Mitotracker staining (Figure 1 C).

While maturing red cells lose their nucleus by expulsion, mitochondria are removed through mitophagy, a variant of autophagy whereby mitochondria are first connected with and then engulfed into autophagosomes (30, 31). When WT and Kap1 KO CD71+Ter119+ cells were compared for the expression of genes associated with this process, it was found that several of them were downregulated in the absence of KAP1, including Nix/Bnip3L, which encodes for an outer mitochondrial membrane protein essential for the autophagic maturation of red cells, as well as Ulk1, GABARAP, sh3glb1, Beclin1 and Bcl2l1 (Figure 2A).

These results show that removal of KAP1 is associated with a failure to induce mitophagy-related gene expression in erythroblasts.

### Example 2: KAP1 promotes red cell mitophagy via the stage-specific repression of micro RNAs

Since the KRAB/KAP1 pathway is mostly known to induce transcriptional repression, it seemed likely that the observed lack of induction of mitophagy effectors was an indirect effect. The miRNA expression profile of control and Kap1 KO CD71+Ter119+ cells were then compared. Amongst 455 miRNAs tested, only 5 were downregulated and 11 upregulated more than two-fold in KO cells. Using a recently described in silico approach (Marin and Vanicek, 2012, PLoS One 7, e32208 *;* Marin and J. Vanicek, 2011, Nucleic acids research 39, 19), potential effector target pairs were identified: several of these miRNAs had mitophagy-associated deregulated transcripts as their targets, notably miR-351, predicted to act on Bnip3L (Figure 2A). Consistent with this hypothesis, levels of miR-351 abruptly dropped in CD71+Ter119+ cells, compared to their CD71+Ter119- precursors, mirroring the previously described (Aerbajinai et al, 2003, Blood 102, 712) induction of Bnip3L transcripts at this stage of erythroid differentiation (Figure 2B).

MiR-351 is encoded as part of a miRNA gene cluster located on chromosome X. Interestingly, miR-503 and miR-322*, the other two members of this cluster, were also upregulated in Kap1 KO erythroblasts (fold changes 2.46 and 2.17 respectively). Thus, chromatin immunoprecipitation coupled to DNA sequencing (ChIPSeq) was used to determine whether KAP1 was recruited to this region in CD71+Ter119+ erythroblasts. This led to the detection of a strong KAP1 peak less than 4kb from the miR-351/503/322* genes (not shown).

To confirm that KAP1 regulates erythropoiesis through the stage-specific repression of miRNAs targeting mitophagy-associated transcripts, the mouse erythroleukemia MEL cell line was used, which is blocked at the proerythroblast stage but can be chemically induced to resume erythroid differentiation (Marks and Rifkind, 1978, Annu Rev Biochem 47, 419). Knockdown of Kap1 (Figure 3A) resulted in decreased rates of erythroid differentiation of MEL cells, correlating with an upregulation of miR-351, a downregulation of Bnip3L and an increase in their mitochondrial content (Figure 3B-E). Furthermore, MEL cells overexpressing miR-351 displayed decreased levels of Bnip3L with a corresponding increase in Mitotracker score (Figure 4A-B). Furthermore, transduction of MEL cells with a GFP-expressing lentiviral vector containing the Bnip3L 3'UTR sequence predicted to contain the miR-351 target 3' of GFP resulted in a miR-351-dependent reduction in GFP expression, compared with cells transduced with a control vector (Figure 4C). Finally, complementation of miR-351-overexpressing MEL cells with a lentiviral vector encoding Bnip3L from a cDNA devoid of this 3'UTR sequence reversed their increase in mitochondrial content (Figure 4D).

These results show that KAP1 regulates erythropoiesis through the stage-specific repression of miR-351 which targets Bnip3L.

### Example 3: Stage-specific KRAB ZFPs mediate KAP1-dependent mitophagy in erythroblasts

Because KAP1 is not a DNA binding protein, an implication of these results was that it might be tethered to the miR-351 and other relevant loci by stage-specific KRAB-ZFPs. Thus, a KRAB-ZFP expression analysis was performed on a large series of hematopoietic cell populations purified by FACS, including hematopoietic stem cells, early and late precursors, and differentiated products of the myeloid, lymphoid and erythroid lineages, quantifying transcripts from 304 murine KRAB-ZFPs and 25 control genes with a custom probe set for the NanoString nCounter platform (Geiss et al., 2008, Nat Biotechnol 26, 317). Nine KRAB-ZFP genes were identified, which both had a human orthologue and were expressed exclusively in CD71+Ter119- and/or CD71+Ter119+ erythroblasts (not illustrated). When each one of these genes was individually knocked down in MEL cells by lentivector-mediated RNA interference, two of them, ZFP689 and ZFP13, emerged as potential Bnip3L regulators, based on transcriptome analyses and Mitrotracker scoring (not illustrated). Interestingly, ZFP689 is expressed in CD71+Ter119- basophilic erythroblasts, whereas ZFP13 is expressed only in their CD71+Ter119+ orthochromatic counterparts (Figure 5A), suggesting that these KRAB-ZFPs cooperate to promote mitophagy at these two consecutive stages of erythroblast differentiation.

To determine whether ZFP689 and ZFP13 interact with the miR-351 locus, a reporter lentiviral vector containing a 500 bp fragment from the X chromosome encompassing the corresponding KAP1-binding site was designed, cloned upstream of a human phosphoglycerate kinase promoter (hPGK) driving the murine secreted alkaline phosphatase (mSEAP), and used this vector to transduce control, ZFP689- or ZFP13-overexpressing MEL cells. SEAP production was decreased in the presence of either KRAB-ZFP, suggesting that both were capable of recognizing the miR-351 locus fragment (Figure 5B).

These two candidates were then validated *in vivo.* For this, bone marrow chimeric mice were generated by transplanting CD45.2 hematopoietic stem cells (lineage-negative, Scal-positive and cKit-positive, also called LSK) transduced with lentiviral vectors producing GFP and shRNAs against ZFP689, ZFP13, or KAP1 as a control, into irradiated CD45.1 animals, allowing the dual discrimination of donor versus recipient and transduced versus untransduced cells. Analyses of the red cell compartment in bone marrow harvested eight weeks after the graft revealed that knockdown of either ZFP689 or ZFP13 led to a strong decrease in CD71+Ter119- cells, as pronounced as that observed with the KAP1 knockdown (Figure 5C). Furthermore, RNA analyses of sorted transduced CD71+Ter119+ cells demonstrated that ZFP689-, ZFP13- and KAP1-depleted cells all exhibited an upregulation of miR-351 (Figure 5D) and a marked downregulation of Bnip3L (Figure 5E).

These results show the erythroblast-specific KRAB-ZFPs control of the miR-351/Bnip3L/mitophagy axis.

### Example 4: A KAP1/miRNA mitophagy regulatory cascade in human erythropoiesis

In a last series of experiments, it was determined whether the hereby-described erythropoiesis-regulating system had its equivalent in humans. For this, firstly, human erythroleukemia (HEL) cells were used, following the same experimental approach as in MEL. Kap1 knockdown (Figure 6A) induced a decrease in the differentiation of HEL cells (Figure 6B) and a rise in their mitochondrial content, which could be rescued by complementation with a shRNA-resistant Kap1 allele (Figure 6C). RNAseq analysis of control versus Kap1 KD cells further revealed that depleting the corepressor resulted in a failure to induce several factors of the mitophagy pathway, amongst which Nix, the human homologue of Bnip3L (Figure 6D).

Murine miR-351 has no direct human orthologue, but sequence analysis allowed determine that hsa-miR-125a-5p and hsa-miR-125b-5p have the same seed. Interestingly, Kap1 knockdown induced an upregulation of hsa-miR-125a-5p in HEL cells, as measured by Nanostring (Figure 6D). Confirming that hsa-miR-125a is a functional orthologue of miR351, its overexpression triggered a downregulation of the mitophagy mediator Nix and a rise in the mitochondrial content of HEL cells (Figure 6E).

As the final demonstration of the role of a KAP1/hsa-miR125a/Nix regulatory cascade in red cell mitophagy, the knockdown of Kap1 was induced in human CD34+ cells isolated from cord blood. These cells can undergo erythroid differentiation, monitored by the expression of the lineage-specific cell surface marker CD235a, also known as glycophorin A, upon exposure to cocktails of cytokines including stem cell factor (SCF), interleukin-3 (IL-3), and erythropoietin (EPO). Using this protocol, it was found that Kap1 knockdown induced a decrease in erythroid differentiation of these cells, correlating with a downregulation of Nix and a rise of their mitochondrial content (Figure 6F), and that this phenotype could also be induced by hsa-miR-125a overexpression (Figure 6G).

These results show that KAP1 controls mitophagy during human erythropoiesis.

In summary, these results unveil a multilayered transcription regulatory system, where protein- and RNA-based repressors are super-imposed in combinatorial fashion to govern the timely triggering of an essential step of eryhropoiesis. These data indicate that ZFP13 and ZFP689 most likely act sequentially to silence miR-351, a microRNA directed at Bnip3L, a key effector of mitophagy. However, it was also observed that several other microRNAs with predicted targets in the mitophagy pathway were upregulated in Kap1-deleted murine erythroblasts. This apparent redundancy, or rather addition of parallel effects aimed at a same physiological process, is commonly observed with RNA interference. The present demonstration that it can be further modulated by KRAB-ZFP-mediated repression, and that the latter can itself be multifactorial, adds a remarkable level of modularity to this type of regulation.

First identified in the mouse, the hereby-described regulatory cascade is conserved in humans, where Kap1 knockdown in erythroleukemia or hematopoietic stem cells results in defective mitophagy and blocked erythropoiesis, due to the persistent repression of Nix by has miR-125a-5p, a human microRNA orthologous to mouse miR-351. ZNF205 and ZNF689, the respective human orthologues of murine ZFP13 and ZFP689, are expressed in HEL cells and induced upon erythroid differentiation of CD34+ cells (not illustrated). Therefore, polymorphism or mutations in any genetic component of the pathway unveiled here, whether ZNF205 and ZNF689, the genomic binding sites of their products, hsa-miR-125a-5p and other KAP1-regulated miRNA genes, or the sequences targeted by these RNA regulators, could underlie phenotypes such as anemia, polycythemia or erythroleukemia.

### List of sequences

Nucleic acid sequence of hsa-miR-125a-5p (homo sapiens)
   SEQ ID NO: 1: ucccugagacccuuuaaccuguga
Nucleic acid sequence of hsa-miR-125b-5p (homo sapiens)
   SEQ ID NO: 2: ucccugagacccuaacuuguga
Nucleic acid sequence of mmu-miR-351 (mus musculus)
   SEQ ID NO: 3: ucccugaggagcccuuugagccug
Nucleic acid sequence of mmu-miR-125a-5p (mus musculus)
   SEQ ID NO: 4: ucccugagacccuuuaaccuguga
Nucleic acid sequence of mmu-miR-135a* (mus musculus)
   SEQ ID NO: 5: uauagggauuggagccguggcg
Nucleic acid sequence of mmu-miR-149 (mus musculus)
   SEQ ID NO: 6: ucuggcuccgugucuucacuccc
Nucleic acid sequence of mmu-miR-338-3p (mus musculus)
   SEQ ID NO: 7: uccagcaucagugauuuuguug
Nucleic acid sequence of mmu-miR-683 (mus musculus)
   SEQ ID NO: 8: ccugcuguaagcuguguccuc
Nucleic acid sequence of mmu-miR-574-3p (mus musculus)
   SEQ ID NO: 9: cacgcucaugcacacacccaca
Nucleic acid sequence of mmu-miR-467d* (mus musculus)
   SEQ ID NO: 10: auauacauacacacaccuacac
Nucleic acid sequence of mmu-miR-467a* (mus musculus)
   SEQ ID NO: 11: cauauacauacacacaccuaca
Nucleic acid sequence of mmu-miR-669f (mus musculus)
   SEQ ID NO: 12: cauauacauacacacacacguau
Amino acid sequence of human ZNF205 (homo sapiens) 
SEQ ID NO: 13:
MSADGGGIQDTQDKETPPEVPDRGHPHQEMPSKLGEAVPSGDTQESLHIKMEP
EEPHSEGASQEDGAQGAWGWAPLSHGSKEKALFLPGGALPSPRIPVLSREGRTR
DRQMAAALLTAWSQMPVTFEDVALYLSREEWGRLDHTQQNFYRDVLQKKNG
LSLGFPFSRPFWAPQAHGKGEASGSSRQAGDEKEWRGACTGAVEVGQRVQTSS
VAALGNVKPFRTRAGRVQWGVPQCAQEAACGRSSGPAKDSGQPAEPDRTPDA
APPDPSPTEPQEYRVPEKPNEEEKGAPESGEEGLAPDSEVGRKSYRCEQCGKGF
SWHSHLVTHRRTHTGEKPYACTDCGKRFGRSSHLIQHQIIHTGEKPYTCPACRK
SFSHHSTLIQHQRIHTGEKPYVCDRCAKRFTRRSDLVTHQGTHTGAKPHKCPIC
AKCFTQSSALVTHQRTHTGVKPYPCPECGKCFSQRSNLIAHNRTHTGEKPYHCL
DCGKSFSHSSHLTAHQRTHRGVRPYACPLCGKSFSRRSNLHRHEKIHTTGPKAL
AMLMLGAAAAGALATPPPAPT
Amino acid sequence of human ZNF689 (homo sapiens) 
SEQ ID NO: 14:
MAPPSAPLPAQGPGKARPSRKRGRRPRALKFVDVAVYFSPEEWGCLRPAQRAL
YRDVMRETYGHLGALGCAGPKPALISWLERNTDDWEPAALDPQEYPRGLTVQ
RKSRTRKKNGEKEVFPPKEAPRKGKRGRRPSKPRLIPRQTSGGPICPDCGCTFPD
HQALESHKCAQNLKKPYPCPDCGRRFSYPSLLVSHRRAHSGECPYVCDQCGKR
FSQRKNLSQHQVIHTGEKPYHCPDCGRCFRRSRSLANHRTTHTGEKPHQCPSC[?]
RRFAYPSLLAIHQRTHTGEKPYTCLECNRRFRQRTALVIHQRIHTGEKPYPCPD[?]
ERRFSSSSRLVSHRRVHSGERPYACEHCEARFSQRSTLLQHQLLHTGEKPYPCP
DCGRAFRRSGSLAIHRSTHTEEKLHACDDCGRRFAYPSLLASHRRVHSGERPYA
CDLCSKRFAQWSHLAQHQLLHTGEKPFPCLECGRCFRQRWSLAVHKCSPKAPN
CSPRSAIGGSSQRGNAH
Amino acid sequence of murine ZFP13 (mus musculus 
SEQ ID NO: 15:
MSADSQSIAATENEEKSHEVPGNVQHCGDMLSGQEETVPLGTSQESTHIKAEPE
EPHSEGASREDRTPGTQRWMSPCPGPKDKVVPSPWTPVLSRGGRTRERKMAA
ALLTAWSQMPVTFEDVALYLSQEEWGRLDHTQQNFYRDVLQGKNGLALGAV
EMGKVVPALAVATLEDTKSIRTRARWAPGEDPKCGQHVASGPGTKLTRDTGK
AGQLKPAPSESRPLKTPEDSGPEKPSEGEEALKSGEEGLVPDGDTGKKTYKCEQ
CGKGFSWHSHLVTHRRTHTGEKPYTCTDCGKRFGRSSHLIQHQIIHTGEKPYTC
PSCWKSFSHHSTLIQHQRIHTGEKPYVCDRCAKRFTRRSDLVTHQGTHTGAKPH
KCPICSKCFTQSSALVTHQRTHTGVKPYPCPECGKCFSQRSNLIAHNRTHTGEKP
YHCLDCGKSFSHSSHLTAHQRTHRGVRPYSCPLCGKSFSRRSNLHRHEKIHTTG
PKALAMLMLGAAAAGALTAPPPAST
Amino acid sequence of murine ZFP689 (mus musculus) 
SEQ ID NO: 16:
MAPPSAPLLLRAVGEAGPTRKRGRRPRALKFVDVAVYFSSEEWGCLQPAQRTL
YRDVMRETYGLLGALGCAGPKPALISWLERNTDDWEPAALDPQEYRRWVTFQ
RKTRSKQKTEEKDVFPPKEAPRKGKRGRKPSKPRLIPRQTSGGPICPDCGCTFPD
HLALESHKCAQNLKKPYPCPDCGRRFSYPSLLVSHRRAHSGECPYVCDQCGKR
FSQRKNLSQHQVIHTGEKPYHCPDCGRCFRRSRSLANHRTTHTGEKPHQCPSCG
RRFAYPSLLAIHQRTHTGEKPYTCLECSRRFRQRTALVIHQRIHTGEKPYPCPDC
ERRFSSSSRLVSHRRVHSGERPYACENCEARFSQRSTLLQHQLLHTGEKPYPCP
DCGRAFRRSGSLAIHRSTHTEEKLHACDDCGRRFAYPSLLASHRRVHSGERPYA
 
CDLCSKRFAQWSHLSQHQLLHTGEKPFPCLECGRCFRQRWSLAVHKCCPNTPN
GSPRPLIGGPSQRSSAL
Nucleic acid sequence of human ZNF205 (homo sapiens) 
SEQ ID NO: 17
atgtctgcagacggcggaggcatccaggacacccaggacaaggagacacccccggaggttccagatcgtggacatcctca
tcaggaaatgccttctaagctgggggaggcggtaccttcaggggacactcaggagtcactgcacattaagatggagcccgaa
gagccacactccgagggggcatcgcaggaggatggggctcaaggtgcctggggctgggcacccctaagtcacggctctaa
ggagaaagctctcttcctgcctggcggagccctcccctcccccggatccccgtgctttcccgagaggggaggaccagaga
ccggcagatggctgcagcgctcctcactgcctggtcccagatgccagtgactttcgaggatgtggccttgtacctctcccggg
aggagtggggacggctggaccacacgcagcagaacttctacagggatgtcctgcagaagaaaaatgggctgtcactgggct
ttcccttcagcaggcctttctgggcccctcaagcgcacggcaagggtgaggcctcgggctccagccggcaggcaggagatg
agaaggagtggagaggcgcgtgcacaggagccgtcgaggtggggcagagggtgcagacctcatccgtggcagcccttgg
gaatgtgaagcccttcagaaccagggcagggagagtccagtggggcgtcccgcagtgcgcgcaggaagcagcctgcggc
cggagctcagggccggccaaagactccgggcagccggctgagccagatcgcaccccggatgcagctccgccagacccc
agtcccacggagccccaggagtaccgcgtcccggagaagcccaacgaggaggagaagggcgccccggagagtggcga
ggagggcctggcccctgacagtgaggtgggcaggaagagctaccggtgcgagcagtgcggcaagggcttcagctggcac
tcgcacctggtgacgcaccggcgcacgcacacgggcgagaagccctacgcctgcactgactgcgggaagcgcttcggcc
gcagctcgcacctcatccagcaccagatcatccacacgggcgagaagccctacacctgccccgcctgccggaagagcttca
gccaccactccacgctgattcagcaccagcgcatccacaccggagagaagccctacgtgtgcgaccgctgcgccaagcgc
ttcacccgccgctcggacttggtcacccaccagggcacccacacgggcgccaagccgcacaagtgccccatctgcgccaa
gtgcttcacgcagagctcggcgctagtcacccaccagcgcacccacactggggtcaagccctatccgtgccccgagtgcgg
caagtgcttcagccagcgttccaacctcatcgcgcacaaccgcacacacacaggcgagaagccctaccactgcctcgactg
cggcaagagcttcagccacagctcgcacctcaccgcgcaccagcgcacccaccgtggcgtgcggccctacgcctgcccgt
tgtgcggcaagagcttcagccggcgctccaacctgcaccggcacgagaagatccacaccaccgggcccaaggccctggc
catgctgatgctgggggcggcggcggcgggggctctggccacacccccacccgctcccacctag
Nucleic acid sequence of human ZNF689 (homo sapiens) 
SEQ ID NO: 18
atggcgccaccttcggctccgctccctgcgcagggaccaggaaaggccagacccagtcggaaaaggggcaggaggccg
agggctctgaagttcgtggacgtggccgtgtacttctccccggaggagtggggctgcctgcggcccgcgcagagggccctg
taccgggacgtgatgcgggagacctacggtcacctgggcgcgctcgggtgcgcaggtcccaaaccagccctcatctcctgg
ttggaacgaaacaccgatgactgggaaccggctgctctagatccgcaggagtacccgagagggctaacagtccagagaaa
aagcagaaccagaaagaagaatggggagaaggaagtattcccgcctaaggaggcaccccgaaaggggaagcgaggccg
gaggcccagcaaaccccgactgattcctaggcagacgtccgggggccccatctgccctgactgcggctgtaccttccctgat
catcaggccctggagagccacaagtgcgcccagaatctaaaaaagccttacccttgcccagactgtgggcgccgcttttccta
tccatccctgctggtcagtcaccggcgggcacactccggcgagtgcccctatgtttgtgaccagtgtggcaaacgtttctccca
gcgcaagaacctctcccagcaccaggtcatccatacaggggagaagccctatcactgccctgactgtggtcgctgcttccgg
aggagccggtccttggccaatcaccggaccacacacacaggtgaaaaaccccaccagtgccctagctgtggacgtcgcttc
gcctacccctccctgctagccatccaccagcgtacacacacgggagagaagccctacacttgcctcgagtgcaaccgccgct
tccgccagcgcacggccctcgtcatccaccagcgcatccacacgggcgagaagccctacccgtgcccggactgcgagcg
gcgcttctcctcctctcgcctggtcagtcaccggcgtgtgcactctggggagcgtccctatgcctgcgagcactgtgaggc
ccgcttctcccagcgcagcacgctgctccagcaccagctcttgcacaccggagagaagccctacccctgcccagactgtgg
gcgtgccttccggcggagcggctccctggccatccatcgcagcacgcacacagaggagaagctgcacgcctgcgacgact
gtggtcgccgctttgcctacccctcactgctggccagccaccggcgcgtgcactcgggcgagcggccctatgcctgcgacct
ttgctccaagcgttttgctcagtggagccacctggcccagcaccagctgctgcacacgggggagaagcctttcccctgcctcg
agtgtggccggtgcttccgccagaggtggtctctggctgtccacaagtgtagccccaaggccccaaactgtagccctagatct
gctatcgggggctccagtcagaggggcaacgcccattag
Nucleic acid sequence of mouse ZFP13 (mus musculus) 
SEQ ID NO: 19
atgtctgcagacagtcaaagcatcgcggctactgagaacgaggagaaatcccatgaggtcccaggtaatgtgcaacactgtg
gagacatgctctctggacaggaggagacagtgcccttgggaacctctcaggagtcaacacacatcaaggcagagccagaa
gagccacacagtgagggggcatcacgggaggacaggactccaggaacacagcgctggatgtccccgtgtcctggccct
aaggacaaagttgttccttctccctggactcctgtactgtcccgagggggacggaccagagagcgaaagatggctgctgcac
tccttactgcctggtcccagatgccggtgacctttgaggatgtggccctgtatctctcccaggaggagtggggaaggctggac
cacacacagcaaaatttctacagagatgtactacaggggaaaaacgggctggctttgggagccgtggaaatggggaaggtg
gttccagccttggccgtcgcaaccctagaggacacaaaatcgataaggactagggccaggtgggcccgggggaagaccc
aaagtgtggtcagcacgtggctagtggcccaggtacaaagctgaccagagacactgggaaagctggtcagctaaagccag
ccccatctgaatcacgtcctctcaagaccccagaggacagcggcccagagaaacccagtgagggggaggaggctctgaaa
agtggcgaagagggtctggtacccgacggtgacacgggcaagaagacatacaagtgcgagcagtgtggcaagggcttca
gttggcactcgcacctggtgacacaccggcgtacgcacacgggcgagaagccttatacctgcacggactgtggcaagcgct
tcggccgcagctcacacctcatccagcaccagatcatccacacgggcgagaagccctatacctgcccctcgtgttggaagag
cttcagccaccactccacgctgatccagcaccagcgcatccacactggtgagaagccctacgtgtgcgaccgctgtgccaag
cgcttcacccgtcgctcggacctagtcacacaccagggcactcacaccggcgccaagccccacaagtgccccatctgcagc
aagtgcttcacccagagctccgccctggtcacgcaccagcgcacccacaccggcgtcaagccctacccgtgcccggagtg
cggcaagtgcttcagccagcgctccaatctcatcgcacataatcgcacccacacgggcgagaagccctaccactgcctcgac
tgtggcaagagcttcagccacagctcgcacctcactgcccaccaacgcactcaccgtggcgtgaggccctactcctgcccac
tttgcggcaagagcttcagccgccgctccaacctgcaccggcacgagaagatccacaccactggacccaaggctctggcca
tgctcatgctgggggcggcagctgcgggggcgctcacggcaccaccacctgcttccacttag
Nucleic acid sequence of mouse ZFP689 (mus musculus)

SEQ ID NO: 20
atggcgccaccttcggctccgcttctcctgcgggcagtcggagaggctggacctactcggaaaaggggcaggaggccaag
ggccttgaagttcgtggacgtggccgtgtacttctcctcggaggagtgggggtgtctgcagcccgctcagaggactctgtacc
gggatgtgatgcgcgagacctacggcctcctgggcgcgctcggatgcgcaggtcccaaaccagctctcatctcctggttgga
acgaaataccgatgactgggaaccagcagccctagatccgcaggagtaccggagatgggtaacattccagagaaaaacca
gatccaagcagaagactgaagagaaggacgtattcccacctaaggaagcaccccgaaaggggaaacgagggcggaagc
ccagcaaaccccggctgattcccaggcagacatccgggggccccatctgccctgactgtggctgtacctttcctgaccacctc
gccctggagagccacaagtgcgcccagaatctgaagaaaccctatccctgcccggattgtgggcgtcgcttttcctacccatc
cctgttggtcagtcaccggcgggcacactccggtgagtgcccttacgtttgtgaccagtgtggcaaacgtttttcccagaggaa
aaacctgtctcagcaccaggtcatacacacaggcgagaagccctaccactgccctgactgtggccgctgctttcgcaggagc
cggtcgttggccaatcaccggaccacgcacaccggtgagaagcctcatcagtgccctagttgtgggcgtcgcttcgcctatcc
atctctgctcgctatccaccaacggacacacacaggggagaagccctatacctgcctagagtgcagccgtcgcttccgccag
cgcaccgccctggtcatccaccagcgcatccatactggagagaagccttacccatgtcctgactgtgagcggcgtttctcctcc
tcctctcgcctggttagccaccggcgtgtgcactctggggagcggccctatgcctgtgagaactgcgaagcccgcttttctcag
cgcagcaccctgctccagcaccagctcttgcacacaggcgagaaaccctacccctgcccagactgtggacgtgccttccgg
aggagcggctccttggccatccatcgcagcacgcacacggaggagaagctgcacgcctgcgatgactgcggccgccgctt
tgcctatccctcactgctggccagtcatcggcgcgtgcactcaggcgagcggccctatgcctgtgacctttgctccaaacgctt
tgcccagtggagccacttgtctcagcaccagcttctgcacactggggaaaagcctttcccctgccttgagtgtggccggtgctt
ccgccagaggtggtccctggctgtccacaagtgctgccccaacacccccaatggtagccctagacctcttataggagggccc
agccagaggagcagtgctctttag

## Claims

1. An agent or composition comprising at least one of: (i) a micro-RNA targeting a mitophagy-associated gene or a variant thereof, (ii) a vector comprising a nucleic acid encoding said micro-RNA or variant thereof, (iii) an inducer of said micro-RNA, and (iv) an inhibitor of said micro-RNA, for use as a medicament.

2. The agent or composition according to claim 1, wherein said micro-RNA is selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), mmu-miR-669f (SEQ ID NO: 12), and a variant thereof.

3. The agent or composition according to claim 1, wherein said micro-RNA is hsa-miR-125a-5p (SEQ ID NO: 1), mmu-miR-351 (SEQ ID NO: 3), or a variant thereof.

4. The agent or composition according to any one of claims 1 to 3, wherein said inducer is a shRNA that silences a nucleic acid encoding a Kruppel-associated box zinc finger protein (KRAB-ZFP) selected among: human ZNF205 (SEQ ID NO: 13), human ZNF689 (SEQ ID NO: 14), mouse ZFP13 (SEQ ID NO: 15), and mouse ZFP689 (SEQ ID NO: 16).

5. The agent or composition according to any one of claims 1 to 3, wherein said inhibitor is (i) a Kruppel-associated box zinc finger protein (KRAB-ZFP) selected among: human ZNF205 (SEQ ID NO: 13), human ZNF689 (SEQ ID NO: 14), mouse ZFP13 (SEQ ID NO: 15), mouse ZFP689 (SEQ ID NO: 16), and a variant thereof, (ii) a vector over-expressing a KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), and a variant thereof, (iii) an antagomir repressing said micro-RNA expression, or (iv) a sponge vector inhibiting said micro-RNA function.

6. The agent or composition according to claim 5, wherein said inhibitor is an antagomir repressing the expression of hsa-miR-125a-5p (SEQ ID NO: 1) or of mmu-miR-351 (SEQ ID NO: 3), or a sponge vector inhibiting the function of hsa-miR-125a-5p (SEQ ID NO: 1) or of mmu-miR-351 (SEQ ID NO: 3).

7. A pharmaceutical composition comprising: (i) a micro-RNA targeting a mitophagy-associated gene or a variant thereof, (ii) a vector comprising a nucleic acid encoding said micro-RNA or variant thereof, (iii) an inducer of said micro-RNA, or (iv) an inhibitor of said micro-RNA; and a pharmaceutically acceptable carrier.

8. An agent or composition according to any one of claims 1 to 7, for use in the treatment, attenuation, or prevention of a mitochondria-related disease.

9. The agent or composition according to claim 8, wherein said mitochondria-related disease is selected among red blood cells-related diseases including anemia, polycythemia and erythroleukemia, lipid-related diseases including obesity and heart diseases, diabetes, neurodegenerative diseases including Parkinson's disease, Alzheimer's disease and Huntington disease, ischemia or drug-induced tissue injury, mitochondria-mediated tissue injury including hepatic necrosis, liver damage and steatosis, age-related diseases, aging, tumors and cancers and inflammation.

10. The agent or composition according to claim 8, wherein said micro-RNA is selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), mmu-miR-669f (SEQ ID NO: 12), and a variant thereof, or said inducer is a shRNA that silences a nucleic acid encoding a KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), and a variant thereof; for the treatment, attenuation, or prevention of polycythemia or obesity caused by fat deposit in the subject's body.

11. The agent or composition according to claim 8, wherein said inhibitor is (i) a Kruppel-associated box zinc finger protein (KRAB-ZFP) selected among: human ZNF205 (SEQ ID NO: 13), human ZNF689 (SEQ ID NO: 14), mouse ZFP13 (SEQ ID NO: 15), mouse ZFP689 (SEQ ID NO: 16), and a variant thereof, (ii) a vector over-expressing a KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), and a variant thereof, (iii) an antagomir repressing said micro-RNA expression, or (iv) a sponge vector inhibiting said micro-RNA function; for the treatment, attenuation, or prevention of anemia, erythroleukemia, neurodegenerative disorders such as Parkinson's disease, Alzheimer's disease and Huntington disease, inflammation caused by damaged mitochondria in cardiomyocytes or preventing development of a heart disease

12. An *ex-vivo* method of prognosis and/or diagnosis of a mitochondria-related disease in a subject comprising determining, in a biological sample from said subject:
a) the level of at least one of: (i) a micro-RNA targeting a mitophagy-associated gene, (ii) a KRAB-ZFP selected among: human ZNF205 (SEQ ID NO: 13), human ZNF689 (SEQ ID NO: 14), mouse ZFP13 (SEQ ID NO: 15), and mouse ZFP689 (SEQ ID NO: 16), (iii) a mitophagy-associated gene, and/or
b) the presence of a nucleic acid comprising a mutation in at least one of: (i) a micro-RNA targeting a mitophagy-associated gene, (ii) a gene encoding a KRAB-ZFP selected among: human ZNF205 (SEQ ID NO: 13), human ZNF689 (SEQ ID NO: 14), mouse ZFP13 (SEQ ID NO: 15), and mouse ZFP689 (SEQ ID NO: 16), (iii) a nucleic acid sequence selected among: human nucleic acid ZNF205 (SEQ ID NO: 17), human nucleic acid ZNF689 (SEQ ID NO: 18), mouse nucleic acid ZFP13 (SEQ ID NO: 19), mouse nucleic acid ZFP689 (SEQ ID NO: 20), (iv) the nucleic acid sequence of the DNA target of at least one KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), (v) a mitophagy-associated gene.

13. The method of claim 12, comprising:
a) Providing a biological sample from said subject;
b) Determining the level of at least one micro-RNA selected among: hsa-miR-125a-5p (SEQ ID NO: 1), hsa-miR-125b-5p (SEQ ID NO: 2), mmu-miR-351 (SEQ ID NO: 3), mmu-miR-125a-5p (SEQ ID NO: 4), mmu-miR-135a* (SEQ ID NO: 5), mmu-miR-149 (SEQ ID NO: 6), mmu-miR-338-3p (SEQ ID NO: 7), mmu-miR-683 (SEQ ID NO: 8), mmu-miR-574-3p (SEQ ID NO: 9), mmu-miR-467d* (SEQ ID NO: 10), mmu-miR-467a* (SEQ ID NO: 11), and mmu-miR-669f (SEQ ID NO: 12), in said sample;
c) Comparing the level of step b) with the level of a corresponding micro-RNA in a control sample:
wherein an alteration of the level of said micro-RNA determined in step b) compared to the control sample is indicative of the subject being at risk for developing, or having, a mitochondria-related disease.

14. The method of claim 12, comprising:
a) Providing a biological sample from said subject;
b) Determining the level of at least one KRAB-ZFP selected among: human ZNF205 (SEQ ID NO: 13), human ZNF689 (SEQ ID NO: 14), mouse ZFP13 (SEQ ID NO: 15), mouse ZFP689 (SEQ ID NO: 16), in said sample;
c) Comparing the level of step b) with the level of a corresponding KRAB-ZFP in a control sample:
wherein an alteration of the level of said KRAB-ZFP determined in step b) compared to the control sample is indicative of the subject being at risk for developing, or having, a mitochondria-related disease; and/or

15. The method according to any one of claims 12 to 14, wherein the biological sample is selected among blood, tissue biopsy, urine, saliva.

16. A method for *in vitro* production of red blood cells comprising a step of inducing mitophagy in cells in culture by contacting said cells with an agent or composition comprising at least one inhibitor of a micro-RNA targeting a mitophagy-associated gene or a variant thereof.
